# FASCICULE DE BREVET EUROPEEN

(11) **EP 0 964 852 B1**
(45) Date de publication et mention de la délivrance du brevet: **12.03.2003**
(21) Numéro de dépôt: 98942786.9
(22) Date de dépôt: 26.08.1998
(51) Int. Cl.: C07D 209/42, C07D 209/60, A61K 31/40

(54) **COMPOSES INDOLE-CARBOXYLIQUES ET LEUR UTILISATION COMME COMPOSES PHARMACEUTIQUES**
INDOLECARBONSÄUREDERIVATE UND IHRE VERWENDUNG ALS ARZNEIMITTEL
INDOLECARBOXYLIC COMPOUNDS AND THEIR USE AS PHARMACEUTICAL COMPOUNDS

(30) Priorité: 05.09.1997 FR 9711076
(43) Date de publication de la demande: 22.12.1999
(60) Demande divisionnaire: 03000188.7
(73) Titulaire: L'OREAL, 75008 Paris (FR)
(72) Inventeur: BERNARD, Bruno, F-92200 Neuilly-sur-Seine (FR); GERST, Catherine, F-92600 Asnières (FR); GALEY, Jean-Baptiste, F-93600 Aulnay-sous-Bois (FR); DALKO, Maria, F-91190 Gif-sur-Yvette (FR); PICHAUD, Patrick, F-78140 Vélizy (FR)
(74) Mandataire: Tezier Herman, Béatrice
(86) Numéro de dépôt international: FR9801853
(87) Numéro de publication internationale: WO99012905

(56) Documents cités:
- WO-A-94/22821
- WO-A-97/35572
- WO-A-99/07351
- DE-A- 2 203 542
- HOLT, DENNIS A. ET AL: "Benzophenone- and Indolecarboxylic Acids: Potent Type-2 Specific Inhibitors of Human Steroid 5.alpha.-Reductase" J. MED. CHEM. (1995), 38(1), 13-15 ,1995, XP002068506 cité dans la demande
- KITA, YASUYUKI ET AL: "Total synthesis of discorhabdin C: a general aza spiro dienone formation from O-silylated phenol derivatives using a hypervalent iodine reagent" J. AM. CHEM. SOC. (1992), 114(6), 2175-80 ,1992, XP002068507
- CHEMICAL ABSTRACTS, vol. 83, no. 7, 18 août 1975 Columbus, Ohio, US; abstract no. 58582, NANTKA-NAMIRSKI, PAWEL ET AL: "Derivatives of 2-carbethoxyindole. III. Products of amidation and alkylation of 5-benzyloxy- and 5-methoxy-2-carbethoxyindole" XP002068510 & ACTA POL. PHARM. (1974), 31(5), 569-76 , 1974,
- CHEMICAL ABSTRACTS, vol. 68, no. 5, 29 janvier 1968 Columbus, Ohio, US; abstract no. 21765, KEGLEVIC, DINA ET AL: "Synthesis of serotonin carbon-14 labeled in the ring (5-hydroxytryptamine-3-14C)" XP002068511 & J. LABELLED COMPD. (1967), 3(2), 144-8 CODEN: JLCAAI,1967,
- CHEMICAL ABSTRACTS, vol. 108, no. 1, 4 janvier 1988 Columbus, Ohio, US; abstract no. 5930, PARTSVANIYA, D. A. ET AL: "Indole derivatives. 128. Synthesis and properties of 5,6- and 4,5-(ethylenedioxy)indoles" XP002068512 & KHIM. GETEROTSIKL. SOEDIN. (1986), (12), 1624-8 ,1986,
- CHEMICAL ABSTRACTS, vol. 92, no. 19, 12 mai 1980 Columbus, Ohio, US; abstract no. 163872, SUVOROV, N. N. ET AL: "Some results of studies of synthesis methods and properties of isomeric pyrroloquinolines" XP002068513 & TR. - MOSK. KHIM.-TEKHNOL. INST. IM. D. I. MENDELEEVA (1977), 94, 23-31 ,1977,
- HOSMANE, R. S. ET AL: "Synthesis of benz[e]indole and benz[g]indole carboxaldehydes" J. HETEROCYCL. CHEM. (1974), 11(1), 29-32 ,1974, XP002068508

## Description

La présente invention concerne l'utilisation d'une quantité efficace d'au moins un composé de la famille des indole-carboxyliques pour traiter les désordres liés à une suractivité de la 5α-réductase de type 1, lesdits désordres sont choisis parmi l'acné, l'hirsutisme, la séborrhée, l'alopécie androgénique, les chéloïdes et les adhésions, les carcinomes prostatiques, l'hyperplasie bénigne de la prostate, le syndrome polycystique ovarien, le syndrome prémenstruel, le cancer du poumon chez l'homme, la puberté précoce, la maladie de Fox et Fordyce.

Les androgènes sont des hormones définies comme appartenant à la famille des stéroïdes possédant une structure spécifique.

Les androgènes agissent à de nombreux sites du corps humains et interviennent par là même dans un grand nombre de désordres parmi lesquels on peut citer les carcinomes prostatiques, l'hyperplasie bénigne de la prostate, l'acné, l'hirsutisme, la séborrhée, l'alopécie androgénique, les chéloïdes et les adhésions, le syndrome polycyclique ovarien, le syndrome prémenstruel, le cancer du poumon chez l'homme, la puberté précoce, la maladie de Fox et Fordyce.

Les androgènes sont des substances lipidiques qui traversent facilement les membranes cellulaires. Le mécanisme d'action des androgènes se fait par l'interaction avec un récepteur qui leur est spécifique : le récepteur aux androgènes.

La testostérone apparaît comme l'androgène majeur. Les voies métaboliques des androgènes, de la testostérone en particulier, sont aujourd'hui bien connues.
Une des voies métaboliques de la testostérone est sa conversion par la 5α-réductase en dihydrotestostérone (DHT). La testostérone et la DHT se lient au récepteur aux androgènes, mais la DHT présente une affinité beaucoup plus élevée pour ce récepteur que la testostérone. De plus la liaison DHT/récepteur est beaucoup plus stable que la liaison testostérone/récepteur.

Deux isoformes de la 5α-réductase ont été à ce jour isolées et clonées. La 5α-réductase de type 1 est majoritairement exprimée dans la peau et les différents compartiments du follicule pileux particulièrement dans les kératinocytes de l'épiderme et/ou du follicule, dans les cellules de la papille dermique, la gaine externe du follicule pileux, la glande sébacée et dans les glandes sudoripares.
La 5α-réductase de type 2 est plutôt exprimée dans l'épididyme, les vésicules séminales, la prostate, la peau génitale foetale ou encore la gaine interne du follicule pileux ou dans les fibroblastes de la peau génitale adulte.

La 5α-réductase de type 1 étant majoritairement exprimée dans la peau et les différents compartiments du follicule pileux, le développement d'inhibiteurs de la 5α-réductase de type 1 représente une approche de choix pour traiter les désordres androgéno-dépendants, qu'ils se manifestent au niveau de la peau ou du follicule pileux.

Depuis longtemps on a cherché à développer des inhibiteurs de la 5α-réductase comme traitement des désordres liés aux androgènes. Dans cet ordre d'idée de nombreux composés ont été proposés. A ce jour, deux classes d'inhibiteurs ont été synthétisées : les inhibiteurs stéroïdiens et les inhibiteurs non-stéroïdiens (voir à cet égard l'article de Chen et col. "The 5α-reductase system and its inhibitors" (Dermatology 1996 ; 193 : 177-184)).
Parmi les inhibiteurs stéroïdiens on peut citer les dérivés 6-azasteroides et surtout les dérivés 4-azasteroides, dont le [17β-(N-tert-butylcarbamoyl)-4-aza-5α-an-drostan-1-en-3-one] (finastéride), un inhibiteur spécifique de l'isotype 2 et le 7β-méthyl-4-aza-cholestan-3-one (MK-386) un inhibiteur spécifique de l'isotype 1. L'inconvénient des inhibiteurs stéroïdiens est qu'ils présentent des effets secondaires non-négligeables qui rendent leur utilisation délicate.

Parmi les inhibiteurs non-stéroïdiens on peut citer certains dérivés de l'acide benzoylaminophénoxybutanoique, des benzoquinolines tels que letrans-8-chloro-4-methyl-1,2,3,4,4a,5,6,10b-octahydrobenzo[*f*]quinolin-3-one
(LY191704), le 4-[3-(-[bis(4-isobutylphenyl)methylamino]-benzoyl)-1H-indol-1-yl]butyric acid (FK143), des acides gras polyinsaturés, des cations tels que le cuivre et le zinc, et des dérivés de l'épicatéchine. De même il a été décrit des dérivés d'indole comme par exemple dans les demandes EP-458207, EP-511477, EP-600084, EP-628040, WO-9113060, WO-9302050, WO-9302051, WO-9305019, WO-9316996, WO-9427990, WO-9505375, WO-9523143, JP-07304736, JP-07188164.

La classe des dérivés indole-carboxyliques a été peu décrite. Holt et collaborateurs (J. Med. Chem., 1995, 38: 13-15) ont décrit des molécules qui présentent une forte activité inhibitrice vis-à-vis de la 5α-réductase de type 2.
On peut encore citer les références suivantes:
- Le document DE 22 03 542A décrit un composé de formule (I') pour lequel R'₁, R'₂ et Z' représentent un atome d'hydrogène, X' et Y' forment, pris ensemble, avec les 2 atomes de carbone les portant, un cycle ayant 5 à 7 atomes de carbone;
- Hosemmane *et al*. (J. Heterocycl. Chem. 11(1), 29-32, 1974) ont décrit un composé de formule (I') pour lequel R'₂ et Z' représentent un atome d'hydrogène et R'₁ un radical CH₃, X' et Y' forment, pris ensemble avec les 2 atomes de carbone les portant, un cycle ayant 6 atomes de carbone;
- Kita et al. (J. Am. Chem. Soc., 1992, 114, 2175-2180) ont décrit un composé de formule (l') pour lequel X' est un radical -O-CH₂Phényl et R'₁, R'₂ et Y' représentent un atome d'hydrogène et Z' un radical OCH₃;
- Un Chemical Abstract (vol.92, n°19, 12 mai 1980 abstract n°163872, XP002068513 & TR. - Mosk. Khim.-Tekhnol. Inst. IM. D. I. Mendeleeva (1997), 94, 23-31, 1977) décrit l'acide 3H-pyrrolo-[3,2-f]-quinoline-carboxylique.
- Un Chemical Abstract (vol. 108, n°1, 4 janvier 1988 abstract n°5980, XP002068512 & Khim. Geterotsikl. Soedin. (1986), (12), 1624-8, 1986) décrit l'acide 2,3-dihydro-7H-1,4-dioxino'2,3-elindole-8-carboxylique,

Ainsi, un premier objet de l'invention concerne l'utilisation d'une quantité efficace d'au moins un composé répondant à la formule générale (I) 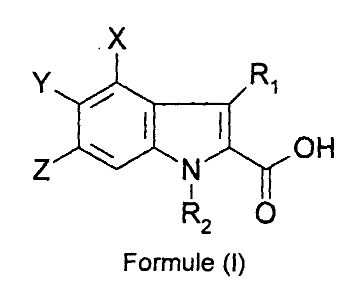 dans laquelle:
X représente un radical -O-CHR₃R₄ dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Y représente un atome d'hydrogène ou un radical -O-CHR₃R₄, dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Z représente un radical -O-R₅ dans lequel R₅ est un radical alkyle en C₁-C₄ choisi parmi les radicaux méthyle, éthyle, propyle, isopropyle, butyle, isobutyle ou tertiobutyle;
ou X et Y forment pris ensemble, avec les 2 atomes de carbone les portant, un cycle ou un hétérocycle ayant 5 ou 6 atomes de carbone et Z représente un atome d'hydrogène ou un radical -O-R₅ dans lequel R₅ est un radical alkyle en C₁-C₄ choisi parmi les radicaux méthyle, éthyle, propyle, isopropyle, butyle, isobutyle ou tertiobutyle;
R₁ représente un atome d'hydrogène ou un radical alkyle en C₁ - C₆ ou un radical aralkyle en C₆ - C₁₂, éventuellement substitué ;
R₂ représente un atome d'hydrogène ou un radical alkyle en C₁ - C₆ ou un radical -CHR₃R₄, dans lequel R₃ et R₄ ont les définitions précédentes ;
étant entendu que lorsque R₃ ou R₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène,
pour la fabrication d'une composition destinée au traitement les désordres liés à une suractivité de la 5α-réductase de type 1, lesdits désordres sont choisis parmi les carcinomes prostatiques, l'hyperplasie bénigne de la prostate, l'acné, l'hirsutisme, la séborrhée, l'alopécie androgénique, les chéloïdes et les adhésions, le syndrome polycystique ovarien, le syndrome prémenstruel, le cancer du poumon chez l'homme, la puberté précoce, la maladie de Fox et Fordyce.

Préférentiellement, les composés répondant à la formule (I) selon l'invention peuvent être utilisés pour induire et/ou stimuler la croissance des cheveux et/ou freiner leur chute et/ou dans le traitement de l'hyperséborrhée et/ou de l'acné.

Préférentiellement, l'invention a pour objet l'utilisation dans une composition, d'une quantité efficace d'au moins un composé répondant à la formule générale (I) tel que défini ci-dessus, ce composé la composition étant destinés à induire et/ou stimuler la croissance des cheveux et/ou freiner leur chute et/ou à traiter l'hyperséborrhée et/ou l'acné.

Bien entendu, les composés répondant à la formule (I) selon l'invention peuvent être utilisés seuls ou en mélange.

Parmi les composés utilisés préférentiellement selon l'invention on peut citer
l'acide 4-benzyloxy-6-méthoxy-1 H-indole-2-carboxylique,
l'acide 4-(3,5-bis-trifluorométhyl-benzyloxy)-6-méthoxy-1 H-indole-2-carboxylique,
l'acide 3H-benzo [E] indole-2-carboxylique,
l'acide 3-méthyl-3H-benzo [E] indole-2-carboxylique,
l'acide 3-benzyl-3H-benzo [E] indole-2-carboxylique,
l'acide 3-(3,5-bis-trifluorométhyl-benzyl)-3H-benzo [E] indole-2-carboxylique,

Parmi ces composés, on préfère tout particulièrement :
l'acide 3H-benzo [E] indole-2-carboxylique,
l'acide 3-méthyl-3H-benzo [E] indole-2-carboxylique.

La quantité de composés de formule (I) utilisable selon l'invention est bien entendu fonction de l'effet recherché et peut donc varier dans une large mesure.

Pour donner un ordre de grandeur, le composé de formule (I) peut être utilisé selon l'invention en une quantité représentant de 0,001% à 10% du poids total de la composition et préférentiellement en une quantité représentant de 0,01% à 5% du poids total de la composition.

Selon l'invention, les composés de formule (I) peuvent être utilisés dans des compositions à usage cosmétique ou pharmaceutique. Préférentiellement selon l'invention, les composés de formule (I) sont utilisés dans des compositions à usage cosmétique

Dans le traitement de la chute des cheveux, comme dans celui de la séborrhée ou de l'acné, la composition cosmétique selon l'invention est à appliquer sur les zones à traiter et est éventuellement laissée en contact plusieurs heures et est éventuellement à rincer. On peut, par exemple, appliquer la composition contenant une quantité efficace d'au moins un composé tel que défini précédemment, le soir, garder celle-ci au contact toute la nuit et éventuellement effectuer un rinçage le matin. Ces applications peuvent être renouvelées quotidiennement pendant un ou plusieurs mois suivant les individus.

Ainsi, la présente invention a pour deuxième objet un procédé de traitement cosmétique des cheveux et/ou du cuir chevelu et/ou la peau, caractérisé par le fait qu'il consiste à appliquer sur les cheveux et/ou le cuir chevelu et/ou la peau, une composition cosmétique comprenant une quantité efficace d'au moins un composé répondant à la formule (I), à laisser celle-ci au contact des cheveux et/ou du cuir chevelu et/ou de la peau, et éventuellement à rincer.

Le procédé de traitement présente les caractéristiques d'un procédé cosmétique dans la mesure où il permet d'améliorer l'esthétique des cheveux et/ou de la peau en leur donnant une plus grande vigueur et un aspect amélioré.

Ainsi, les indole-carboxyliques présentent des activités remarquables qui justifient leur utilisation pour traiter les désordres liés à une suractivité de la 5α-réductase de type 1, lesdits désordres sont choisis parmi l'acné, l'hirsutisme, la séborrhée, l'alopécie androgénique, les chéloïdes et les adhésions, les carcinomes prostatiques, l'hyperplasie bénigne de la prostate, le syndrome polycystique ovarien, le syndrome prémenstruel, le cancer du poumon chez l'homme, la puberté précoce, la maladie de Fox et Fordyce.

A cet égard, après de long travaux la demanderesse a découvert de nouveaux dérivés indole-carboxyliques qui sont des inhibiteurs spécifiques de la 5α-réductase de type 1.

Ainsi, l'invention a pour troisième objet un dérivé d'indole-carboxylique répondant à la formule générale (I') : 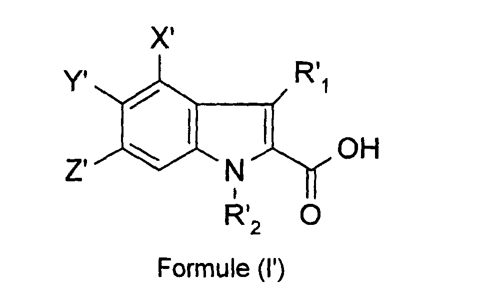 dans laquelle
X' représente un radical -O-CHR'₃R'₄, dans lequel R'₃ est un atome d'hydrogène ou un radical phényle éventuellement substitué ou encore un hétérocycle ayant 5 ou 6 chaînons et R'₄ est un radical phényle éventuellement substitué ou encore un hétérocycle ayant 5 ou 6 chaînons, ou R'₃ et R'₄ pris ensemble forment avec l'atome de carbone un cycle ou un hétérocycle ayant 5 ou 6 chaînons ;

Y' représente un atome d'hydrogène ou un radical -O-CHR'₃R'₄, dans lequel R'₃ est un atome d'hydrogène ou un radical phényle éventuellement substitué ou encore un hétérocycle ayant 5 ou 6 chaînons et R'₄ est un radical phényle éventuellement substitué ou encore un hétérocycle ayant 5 ou 6 chaînons, ou R'₃ et R'₄ pris ensemble forment avec l'atome de carbone un cycle ou un hétérocycle ayant 5 ou 6 chaînons ;
Z' représente un radical -O-R'₅ dans lequel R'₅ est un radical alkyle en C₁-C₄ choisi parmi les radicaux méthyle, éthyle, propyle, isopropyle, butyle, isobutyle ou tertiobutyle;
ou X' et Y' forment pris ensemble, avec les 2 atomes de carbone les portant, un cycle ou un hétérocycle ayant 5 ou 6 atomes de carbone et Z représente un atome d'hydrogène ou un radical -O-R'₅ dans lequel R'₅ est un radical alkyle en C₁-C₄ choisi parmi les radicaux méthyle, éthyle, propyle, isopropyle, butyle, isobutyle ou tertiobutyle;
R'₁ représente un atome d'hydrogène ou un radical alkyle en C₁ - C₆ ou un radical aralkyle en C₆ - C₁₂, éventuellement substitué ;
R'₂ représente un atome d'hydrogène ou un radical alkyle en C₁ - C₆ ou un radical -CHR'₃R'₄, dans lequel R'₃ et R'₄ ont les définitions précédentes ;
ou ses esters ou ses isomères optiques, seuls ou en mélange en toutes proportions, ou encore ses sels pharmaceutiquement acceptables.
étant entendu :
que lorsque R'₁, R'₂ et Z' représentent un atome d'hydrogène, alors X' et Y' ne doivent pas former pris ensemble, avec les 2 atomes de carbone les portant, un cycle ayant 5 à 7 atomes de carbone,
que lorsque R'₂ et Z' représentent un atome d'hydrogène et R'₁ un radical CH₃ alors X' et Y' ne doivent pas former pris ensemble, avec les 2 atomes de carbone les portant, un cycle ayant 6 atomes de carbone,
que lorsque X' est un radical -O-CH₂Phényl et R'₁, R'₂ et Y' représentent un atome d'hydrogène alors Z' ne doit pas être un radical OCH₃,
que le dérivé de formule (I') n'est pas l'acide 3H-pyrrolo-[3,2-f]-quinoline-carboxylique ou l'acide 2,3-dihydro-7H-1,4-dioxino'2,3-elindole-8-carboxylique,
et que lorsque R'₃ ou R'₄ indépendamment l'un de l'autre sont un radical phényle éventuellement substitué, celui-ci est substitué par un groupement cyano (-CN), un groupement trifluorométhyle (-CF₃), un radical méthoxy (-O-CH₃) ou un atome d'halogène.

Par hétérocycle, on entend de préférence selon l'invention un cycle incluant éventuellement un ou plusieurs atomes d'azote et/ou d'oxygène et particulièrement la pyridine, l'imidazole; le tétrahydrofuranne ou le furanne. Un hétérocycle particulièrement préféré selon l'invention est la pyridine.

Par radical alkyle en C₁ - C₆, on entend de préférence selon l'invention les radicaux alkyles linéaires ou ramifiés, saturés ou insaturés, ayant de 1 à 6 atomes de carbone.
Préférentiellement selon l'invention, le radical alkyle est en C₁-C₄ et est choisi parmi les radicaux méthyle, éthyle, propyle, isopropyle, butyle, isobutyle ou tertiobutyle et plus particulièrement les radicaux méthyle ou éthyle.

Par radical aralkyle en C₆ - C₁₂, on entend de préférence selon l'invention les radicaux alkyles-aryles ayant de 6 à 12 atomes de carbone, définition dans laquelle le terme aryle s'entend comme un cycle aromatique ayant 5 ou 6 chaînons ou un hétérocycle aromatique ayant 5 ou 6 chaînons. Préférentiellement selon l'invention, le radical aralkyle est en C₇-C₁₀. Un radical aralkyle particulièrement préféré selon l'invention est le radical benzyle.

Par un radical phényle substitué, on entend selon l'invention le radical phényle substitué par un groupement cyano (-CN), un groupement trifluorométhyle (-CF₃), un radical méthoxy (-O-CH₃) ou un atome d'halogène. L'atome d'halogène peut être choisi parmi le chlore, le brome, le fluor, l'iode. Un radical phényle substitué particulièrement préféré selon l'invention est le radical phényle substitué par un groupement trifluorométhyle (-CF₃).

Selon une forme de réalisation particulière de l'invention, R'₁ est préférentiellement un radical méthyle.

Selon une autre forme de réalisation de l'invention, R'₁ est un radical benzyle.

Selon une forme de réalisation particulière de l'invention, R'₂ est préférentiellement un radical méthyle.

Selon une autre forme de réalisation de l'invention, R'₂ est un radical di-3,5-(trifluorométhyl)benzyle.

Lorsque R'₃ est un radical phényle substitué, R'₃ est préférentiellement un radical di-3,5-(trifluorométhyl)phényle.

Lorsque R'₃ est un hétérocycle, R'₃ est préférentiellement une pyridine.

Lorsque R'₄ est un radical phényle substitué, R'₄ est préférentiellement un radical di-3,5-(trifluorométhyl)phényle.

Lorsque R'₄ est un hétérocycle, R'₄ est préférentiellement une pyridine.

Selon une forme de réalisation particulière de l'invention, R'₅ est préférentiellement un radical méthyle.

Selon une autre forme de réalisation de l'invention, R'₅ est un radical éthyle.

On peut citer comme composés de formule (I') :
l'acide 4-(3,5-bis-trifluorométhyl-benzyloxy)-6-méthoxy-1 H-indole-2-carboxylique,
l'acide 3-méthyl-3H-benzo [E] indole-2-carboxylique,
l'acide 3-benzyl-3H-benzo [E] indole-2-carboxylique,
l'acide 3-(3,5-bis-trifluorométhyl-benzyl)-3H-benzo [E] indole-2-carboxylique.

Parmi ces composés, on préfère tout particulièrement l'acide 3-méthyl-3H-benzo [E] indole-2-carboxylique.

Un quatrième objet de l'invention concerne des compositions cosmétiques ou pharmaceutiques, particulièrement dermatologiques, qui comprennent au moins un des composés répondant à la formule (I') définis ci-dessus.

Bien entendu les compositions selon l'invention peuvent comprendre les composés de formule (I') seuls ou en mélanges en toutes proportions.

La quantité de composés de formule (I') contenue dans les compositions de l'invention est bien entendu fonction de l'effet recherché et peut donc varier dans une large mesure.

Pour donner un ordre de grandeur, la composition de l'invention peut contenir au moins un composé de formule (I') en une quantité représentant de 0,001% à 10% du poids total de la composition et préférentiellement en une quantité représentant de 0,01% à 5% du poids total de la composition.

La composition de l'invention peut être une composition à usage cosmétique ou pharmaceutique. Préférentiellement selon l'invention, La composition de l'invention peut être une composition à usage cosmétique;

La composition peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins composé de formule (I) à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un composé tel que défini précédemment soit sous forme Liposomé, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

Lorsque la composition selon l'invention est une composition pharmaceutique, elle peut être administrée par voie parentérale, entérale ou encore par voie topique. De préférence, la composition pharmaceutique est administrée par voie topique.

Un cinquième objet de l'invention concerne l'utilisation à titre de médicament des composés de formule générale (1').

Les composés de l'invention peuvent être synthétisés par des procédés tout à fait classiques généralement utilisés en synthèse organique.
Des exemples détaillés de ces synthèses sont donnés par ailleurs dans les exemples.
Mais à titre d'exemple et de manière très générale, pour obtenir un composé de l'invention substitué en position 4,6, on fait dans une première étape réagir du 2-hydroxy-4-alkoxy benzaldéhyde de formule 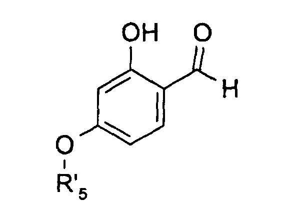 avec un bromure de formule

Br-R

dans laquelle R représente un atome d'hydrogène ou un radical O-CHR'₃R'₄ tel que défini précédemment, et R'₅ est tel que défini précédemment
en présence de tetrahydrofurane (THF) anhydre, d'hydrure de sodium à 60% dans l'huile et d'iodure de tétrabutylammonium.
Après 6 heures d'incubation à température ambiante, le composé obtenu est purifié par chromatographie sur gel de silice.
Par température ambiante on entend un température comprise entre 18°C et 35°C préférentiellement, comprise entre 20°C et 30°C.

Dans une deuxième étape le composé obtenu à la première étape est mis à réagir en présence de méthylate de sodium et d'azido acétate d'éthyle en présence de méthanol pendant 20 heures à température ambiante.
Le composé ainsi obtenu est purifié par chromatographie sur gel de silice ou par recristallisation.

Dans une troisième étape le composé obtenu à la seconde étape est mis à réagir en présence de toluène, à reflux pendant une heure et demi. Le composé ainsi obtenu est purifié par précipitation.

Dans une quatrième étape, le composé obtenu à la troisième étape est mis à réagir en présence de méthanol, d'acétone et de soude pendant 4 heures. Le composé ainsi obtenu est purifié par précipitation.

De même à titre d'exemple et de manière très générale pour obtenir un composé de l'invention substitué en position 5,6, on réalise dans une première étape la nitration du 3-aralcoxy-4-alcoxybenzadehyde de formule 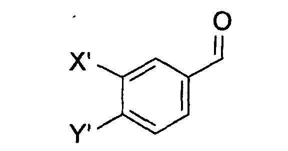 dans laquelle X' et Y' sont définis comme précédemment, par l'acide nitrique à température ambiante, pendant un temps compris entre 2h et 12h.
Le composé obtenu est isolé par filtration.

Dans une deuxième étape le composé obtenu à la 1^{ère} étape est mis à réagir en présence de carbonate de calcium (K₂CO₃) avec du phosphonate de triéthyle dans un mélange d'éthanol et diméthoxyéthane à 80°C pendant un temps compris entre 10 à 20 heures. Le composé obtenu est purifié par chromatographie sur colonne de silice.

Dans une troisième étape le composé obtenu à la 2^{iéme} étape est mis à réagir avec du triéthyle phosphate au reflux pendant un temps compris entre 2 et 6 heures. Le composé obtenu est purifié par chromatographie sur colonne de silice.

Dans une quatrième étape le composé obtenu à la 3^{ième} étape est saponifié par la soude dans un mélange acétone / méthanol pendant un temps compris entre 1 à 6 heures à une température comprise entre 20°C et 40°C. Le composé obtenu est purifié par cristallisation.

Pour obtenir un composé pour lequel X' et Y' forment un cycle ou un hétérocycle on fait dans une première étape réagir un aldéhyde aromatique bicyclique en présence de méthylate de sodium, d'azido acétate d'éthyle, et de méthanol.

Après plusieurs heures d'incubation à température ambiante, le composé obtenu est purifié par chromatographie sur colonne de silice.

Dans une deuxième étape le composé obtenu à la première étape est mis à réagir en présence de toluène, à reflux pendant plusieurs heures. Le composé ainsi obtenu est purifié par recristallisation.

Dans une troisième étape le composé obtenu à la seconde étape est mis à réagir en présence de méthanol, d'acétone et de soude pendant 4 heures.
Le composé ainsi obtenu est purifié par recristallisation.

Pour la préparation des composés dans lesquels R'₂ n'est pas un atome d'hydrogène, le composé obtenu à la seconde étape est mis en présence d'un halogénure d'alkyle pendant 30 minutes à température ambiante. Le composé alors obtenu est ensuite mis à réagir en présence d'acétone et de soude pendant 1 heure à température ambiante. Le composé ainsi obtenu est purifié par recristallisation.

Les indole-carboxyliques présentent des activités inhibitrices de la 5α-réductase qui ont pu être mises en évidence grâce à un test de criblage basé sur l'expression in vitro des isoformes 1 ou 2 de la 5α-réductase.

Les détails de ce tests et les résultats obtenus sont présentés dans les exemples.

On va maintenant donner à titre d'illustration des exemples.

### Exemple n°1 : synthèse de l'acide 4-benzyloxy-6-méthoxy-1H-indole-2-carboxylique

a) Synthèse du 2-benzyloxy-4-méthoxy-benzaldéhyde de formule : 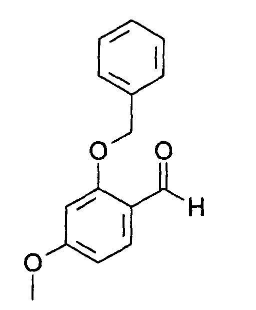
   Dans un réacteur, on solubilise 1 g de 2-hydroxy-4-méthoxy benzaldéhyde dans 15 ml de tétrahydrofuranne (THF) anhydre. On refroidit à 4°C. On ajoute 1,3 équivalents d'hydrure de sodium (NaH) à 60% dans l'huile. On laisse réagir 30 minutes pour former l'alcoolate. On ajoute ensuite 1,3 équivalents de bromure de benzyle, et 90 mg d'iodure de tétrabutylammonium. On laisser réagir pendant 6 heures à la température ambiante.
   Après 6h de réaction, on ajoute 50 ml d'une solution saturée en bicarbonate de sodium (NaHCO₃). On fait deux lavages par 50 ml d'éther isopropylique. On sèche les phases organiques sur sulfate de sodium et évaporation sous vide et on purifie le composé obtenu sur gel de silice en utilisant le dichlorométhane comme éluant. La masse récupérée est de 1,1 g ce qui correspond à un rendement de 69%.
b) Synthèse de l'ester méthylique de l'acide 2-azido-3-(2-benzyloxy-4-méthoxy-phényl)-acrylique de formule : 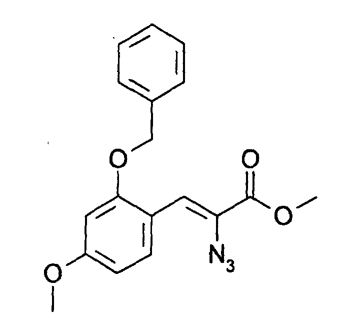
   Dans un tricol parfaitement sec, on introduit 2 équivalents de méthylate de sodium en poudre dans 10 ml de méthanol, le tout sous argon. On verse ensuite 1,1 g de 2-benzyloxy-4-méthoxy benzaldéhyde préalablement solubilisés dans 2 ml de méthanol.
   On refroidit à -10°C. On solubilise 5 équivalents d'azido acétate d'éthyle dans 5 ml de méthanol, et on verse lentement sur la solution de 2-benzyloxy-4-méthoxy benzaldéhyde. On laisse réagir 20 heures après retour à la température ambiante. On dilue le milieu par 100 ml de dichlorométhane, et on fait deux lavages par 50 ml d'eau. On sèche les phases organiques sur sulfate de sodium et évaporation sous vide et on purifie le composé obtenu sur gel de silice en utilisant l'heptane comme éluant (gradient d'acétate d'éthyle (AcOEt) jusqu'à 10%).
   La masse récupérée est de 0,25 g, ce qui correspond à un rendement de 15%.
c) Synthèse de l'ester méthylique de l'acide 4-benzyloxy-6-méthoxy-1H-indole-2-carboxylique de formule : 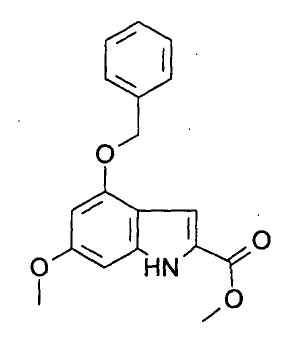
   On solubilise 0,25 g de l'ester méthylique de l'acide 2-azido-3-(2-benzyloxy-4-méthoxy-phényl)-acrylique dans 10 ml de toluène. On porte au reflux. Après 1h30, on concentre le milieu à sec sous vide et on lave le solide obtenu par 20 ml d'heptane. On obtient un solide. La masse récupérée est de 0,11 g, ce qui correspond à un rendement de 50%.
d) Synthèse de l'acide 4-benzyloxy-6-méthoxy-1H-indole-2-carboxylique de formule : 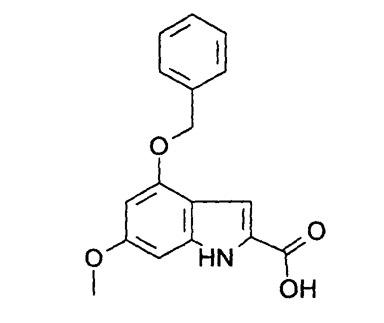
   On solubilise 0,11 g de l'ester méthylique de l'acide 4-benzyloxy-6-méthoxy-1H-indole-2-carboxylique dans 3 ml d'acétone et 2 ml de méthanol. On ajoute ensuite 6 ml de soude 1N. On laisse réagir 4 heures à température ambiante. On concentre le milieu au rotavapor, et on dilue par 20 ml d'eau. On acidifie à pH = 2 par de l'acide chlorhydrique concentré. On obtient un solide marron clair. La masse récupérée est de 700 mg, ce qui correspond à un rendement de 67%.

Les analyses en RMN ¹H (200MHz ; CDCl₃) δ ppm ont donné les résultats suivants :
3.66 (3H ; s), 5.0 (2H ; s), 6.1 (1H ; s), 6.27 (1H ; s), 7.25 (6H ; m), 8.66 (1H ; s) La structure du composé obtenu est conforme à la structure attendue.

### Exemple n°2 : synthèse de l'acide 4-(3,5-bis-trifluorométhyl-benzyloxy)-6-méthoxy-1H-indole-2-carboxylique :

a) Synthèse du 2-(3,5-bis-trifluorométhyl-benzyloxy)-4-méthoxybenzaldéhyde de formule : 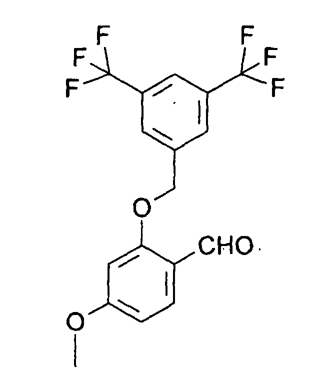
   Dans un réacteur, on pèse 1,1 équivalents d'hydrure de sodium (NaH) en dispersion dans l'huile. On ajoute 20 ml de diméthylformamide (DMF) anhydre. On ajoute ensuite 0,95 équivalent de 2-hydroxy-4-méthoxy benzaldéhyde pour former l'alcoolate. On laisser 30 minutes à température ambiante.
   On ajoute 4,2 g de bromure de (3,5-bis-trifluorométhyle) de benzyle. On laisse réagir 1h30 à température ambiante. On verse 25 ml d'une solution saturée bicarbonate de sodium (NaHCO₃) et on fait deux extractions par 20 ml de dichlorométhane. On sèche les phases organiques sur sulfate de sodium et on les concentre sous vide afin d'obtenir une huile légèrement rosée. On reprend cette dernière dans 15 ml d'heptane, puis dans 20 ml d'éther isopropylique. On obtient 3,6 g de solide blanc cassé, ce qui correspond à un rendement de 74%.
b) Synthèse de l'ester méthylique de l'acide 2-azido-3-[2-(3,5-bis-trifluorométhyl-benzyloxy)-4-méthoxy-phényl]-acrylique de formule : 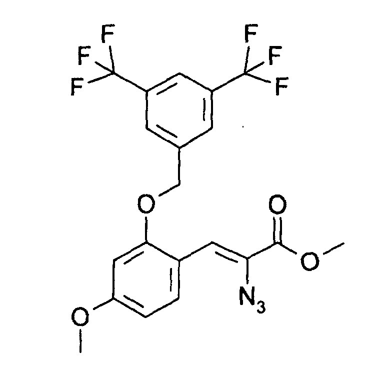
   Dans un réacteur parfaitement séché à l'étuve, on pèse 1 g de méthylate de sodium en poudre. On ajoute 10 ml de méthanol, puis 3,5 g de 2-(3,5-bis-trifluorométhyl-benzyloxy)-4-méthoxy-benzaldéhyde préalablement solubilisés dans 5 ml de méthanol. On refroidit à 0°C.
   On solubilise 4 équivalents d'azido acétate d'éthyle dans 5 ml de méthanol, et on verse ensuite sur le milieu précédent. On laisser réagir au retour à la température ambiante pendant 30 heures.
   On dilue le milieu par 20 ml de dichlorométhane, et on fait deux lavages par 50 ml d'eau. On sèche la phase organique sur sulfate de sodium. On concentre à sec sous vide et on purifie l'huile obtenue sur colonne de silice en utilisant le dichlorométhane comme éluant et en faisant un gradient de méthanol (jusqu'à 2%). La masse récupérée est de 700 mg, ce qui correspond à un rendement de 12%.
c) Synthèse de l'ester méthylique de l'acide 4-(3,5-bis-trifluorométhylbenzyloxy)-6-méthoxy-1H-indole-2-carboxylique de formule : 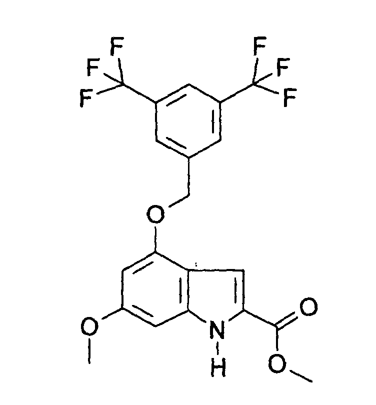
   On solubilise 700 mg de l'ester méthylique de l'acide 2-azido-3-[2-(3,5-bis-trifluorométhyl-benzyloxy)-4-méthoxy-phényl]-acrylique dans 25 ml de toluène. On porte à reflux pendant 3 heures. On concentre le milieu à sec sous vide. On obtient un solide jaune clair. On reprend ce dernier dans 10 ml d'éther isopropylique. La masse récupérée est de 460 mg, ce qui correspond à un rendement de 83%.
d) Synthèse de l'acide 4-(3,5-bis-trifluorométhyl-benzyloxy)-6-méthoxy-1H-indole-2-carboxylique de formule : 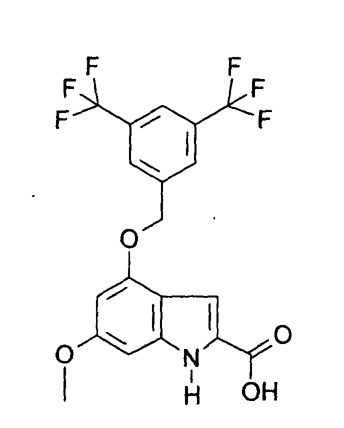
   On solubilise 450 mg de l'ester méthylique de l'acide 4-(3,5-bis-trifluorométhylbenzyloxy)-6-méthoxy-1H-indole-2-carboxylique dans 15 ml d'acétone, puis on ajoute 15 ml de soude 1N. On laisse agiter 4 heures à la température ambiante. On évapore l'acétone sous vide. On dilue le milieu par 10 ml d'eau, et on neutralise à pH = 7 par de l'acide chlorhydrique concentré. On obtient 300 mg de solide blanc ce qui correspond à un rendement de 69%.

Les analyses en RMN ¹H (200MHz ; DMSO) δ ppm ont donné les résultats suivants :
3.75 (3H ; s); 5.42 (2H ; s), 6.28 (1H ; s), 6.51 (1H ; s), 6.95 (1H ; s), 8.1 (1H ; s), 8.2 (2H ; s), 11.5 (1H ; s)
La structure du composé obtenu est conforme à la structure attendue.

### Exemple n°3 : synthèse de l'acide 5-(3,5-bis-trifluorométhyl-benzyloxy)-6-méthoxy-1H-indole-2-carboxylique :

a) Synthèse de l'ester éthylique de l'acide 5-hydroxy-6-méthoxy-1 H-indole-2-carboxylique de formule 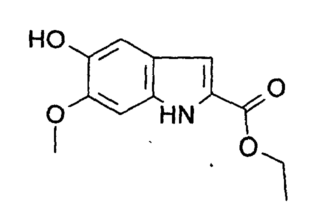
   On solubilise 4,8 g de l'ester éthylique de l'acide 5-benzyloxy-6-méthoxy-1H-indole-2-carboxylique, dans 150 ml d'acétate d'éthyle (AcOEt) et 30 ml d'éthanol. On ajoute 4,8 g de Palladium activé (10%) sur charbon et on effectue la débenzylation sous 500 KPa (5 bars) d'hydrogène pendant 2h30. On filtre le catalyseur sur célite et on concentre à sec le milieu sous vide. On obtient un solide légèrement gris clair.
   La masse récupérée est de 2,55 g, ce qui correspond à un rendement de 72%.
b) Synthèse de l'ester éthylique de l'acide 5-(3,5-bis-trifluorométhylbenzyloxy)-6-méthoxy-1 H-indole-2-carboxylique 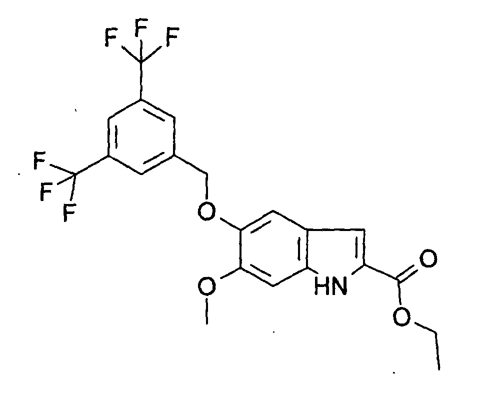
   Dans un réacteur préalablement séché à l'étuve, on introduit 1,1 équivalents d'hydrure de sodium (NaH) sous argon, et 5 ml de diméthylformamide (DMF) anhydre. On ajoute ensuite 0,3 g de l'ester éthylique de l'acide 5-hydroxy-6-méthoxy-1H-indole-2-carboxylique à +3°C, et on laisse réagir 30' à cette température, afin de former l'alcoolate.
   On ajoute ensuite 1,05 équivalents de bromure de 3,5-bis-trifluorométhyle benzyle, toujours à +3°C. On laisse réagir 1h30 à cette température. On ajoute 25 ml d'une solution saturée en bicarbonate de sodium (NaHCO₃) sous agitation pendant 1 heure. On lave deux fois par 50 ml de dichlorométhane. Les phases organiques sont séchées sur sulfate de sodium et concentrées à sec sous vide. Le résidu obtenu est purifié sur colonne de silice en utilisant le dichlorométhane comme solvant.
   La masse récupérée est de 220 mg, ce qui correspond à un rendement de 37%.
c) Synthèse de l'acide 5-(3,5-bis-trifluorométhyl-benzyloxy)-6-méthoxy-1H-indole-2-carboxylique de formule : 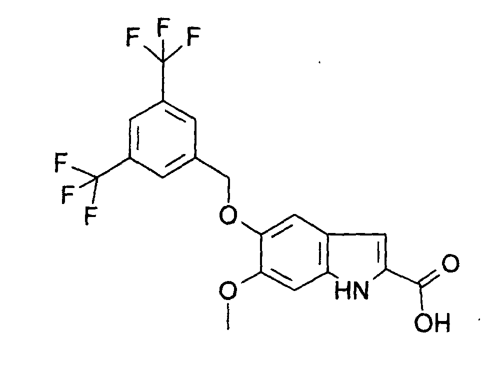
   On solubilise 220 mg de l'ester éthylique de l'acide 5-(3,5-bis-trifluorométhylbenzyloxy)-6-méthoxy-1H-indole-2-carboxylique dans 10 ml d'acétone et 5 ml de méthanol. On ajoute 15 ml de soude 1N, et on laisse réagir 3 heures à 30°C. On concentre les solvants sous vide, puis on dilue par 5 ml d'eau. On acidifie à pH = 2 par de l'acide chlorhydrique concentré. On obtient un précipité que l'on lave par de l'eau. On reprend le solide encore humide dans 50 ml de pentane à reflux pendant 1heure. On filtre sous vide et on obtient 70 mg de solide pour un rendement de 34%.

Les analyses en RMN ¹H (200MHz ; DMSO) δ ppm ont donné les résultats suivants :
3.81 (3H ; s), 5.27 (3H ; s), 5.27 (2H ; s), 6.91 (1H ; s), 6.95 (1H ; s), 7.24 (1H ; s), 8.08 (1H ; s), 8.17 (2H ; s), 11.53 (1H ; s), 12.65 (1H ; s)
La structure du composé obtenu est conforme à la structure attendue.

| Analyse Elémentaire : | | | | |
|---|---|---|---|---|
| | C en % | H en % | N en % | F en % |
| théorique | 52,67 | 3,02 | 3,23 | 26,31 |
| trouvée | 52,09 | 3,11 | 3,11 | 26,44 |

### Exemple n°4 : synthèse de l'acide 5-(3,4,5-triméthoxy-benzyloxy)-6-méthoxy-1H-indole-2-carboxylique :

a) Synthèse de l'ester éthylique de l'acide 5-(3,4,5-triméthoxy-benzyloxy)-6-méthoxy-1H-indole-2-carboxylique de formule : 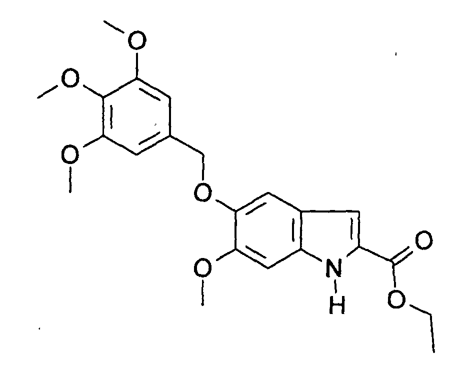
   Dans un réacteur préalablement séché à l'étuve, on introduit 1,1 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1 H-indole-2-carboxylique à +3°C, et on laisse réagir 30 minutes à cette température afin de former l'alcoolate.
   On ajoute ensuite 1,05 équivalents de chlorure de 3,4,5-triméthoxybenzyle, toujours à +3°C. On laisse réagir 1h30 à cette température. On ajoute 10% en mole de iodure de tétrabutylammonium et on laisse réagir 2 heures supplémentaires. On ajoute alors 25 ml d'une solution saturée en bicarbonate de sodium (NaHCO3) sous agitation pendant 1 heure. On lave deux fois par 50 ml de dichlorométhane. Les phases organiques sont séchées sur sulfate de sodium et concentrées à sec sous vide. Le résidu obtenu est purifié sur colonne de silice en utilisant le dichlorométhane comme solvant.
   La masse récupérée est de 200 mg, ce qui correspond à un rendement de 38%.
b) Synthèse de l'acide 5-(3,4,5-triméthoxy-benzyloxy)-6-méthoxy-1H-indole-2-carboxylique de formule : 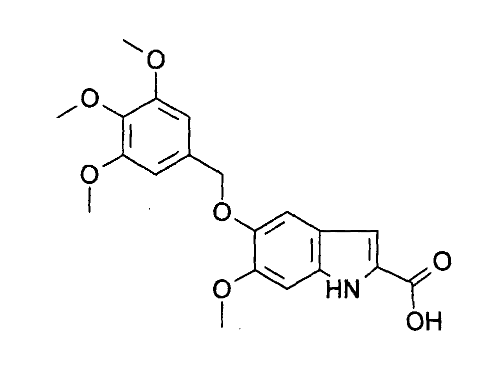
   On solubilise 180 mg de l'ester méthylique de l'acide 5-(3,4,5-triméthoxybenzyloxy)-6-méthoxy-1H-indole-2-carboxylique dans 15 ml d'acétone et 3 ml de méthanol. On ajoute 15 ml de soude 1N, et on laisse réagir pendant 2 heures à 30°C. On évapore les solvants sous vide et on acidifie la phase aqueuse résiduelle à pH = 2, par de l'acide chlorhydrique concentré. On obtient un solide que l'on purifie par reprise dans l'acétate d'éthyle à température ambiante.
   La masse récupérée est de 35 mg, ce qui correspond à un rendement de 21%.

Les analyses en RMN ¹H (200MHz ; CDCl₃) δ ppm ont donné les résultats suivants :
3.6 (3H ; s), 3.85 (9H ; s), 4.95 (2H ; s), 6.7 (1H ; s), 6.8 (2H ; s), 6.9 (1H ; s), 7.5 (1H ; s), 11.05 (1H ; se)
La structure du composé obtenu est conforme à la structure attendue.

### Exemple n°5 : synthèse de l'acide 5-(4-méthoxy-benzyloxy)-6-méthoxy-1H-indole -2- carboxylique :

a) Synthèse de l'ester éthylique de l'acide 5-(4-méthoxy-benzyloxy)-6-méthoxy-1 H-indole-2-carboxylique de formule : 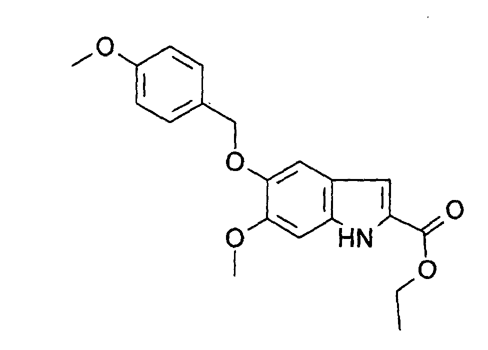
   Dans un réacteur préalablement séché à l'étuve, on introduit 1,1 équivalents d'hydrure de sodium (NaH) sous argon et 5 ml de diméthylformamide (DMF) anhydre. On ajoute ensuite 0,4 g de l'ester éthylique de l'acide 5-hydroxy-6-méthoxy-1H-indole-2-carboxylique à 0°C, et on laisse réagir 30 minutes à cette température, afin de former l'alcoolate. On ajoute ensuite 1,1 équivalents de chlorure de 4-méthoxybenzyle et 10% en mole d'iodure de tétrabutylammonium. On laisse réagir jusqu'au retour à la température ambiante. La réaction est complète après 24 heures. On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide, et on purifie le résidu obtenu sur colonne de silice en utilisant le dichlorométhane comme éluant. La masse récupérée est de 200 mg, ce qui correspond à un rendement de 32%.
b) Synthèse de l'acide 5-(4-méthoxy-benzyloxy)-6-méthoxy-1H-indole-2-carboxylique de formule : 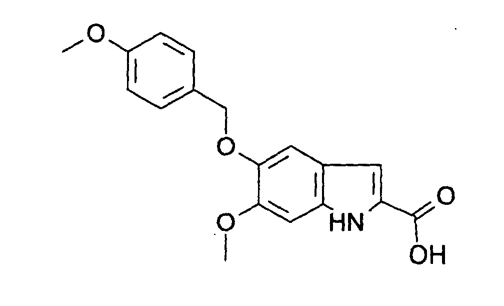
   Dans un réacteur, on solubilise 0,2 g d'ester éthylique de l'acide 6-méthoxy-5-(4-méthoxy-benzyloxy)-1H-indole-2-carboxylique dans 15 ml d'acétone. On ajoute 15 ml de soude 1N, et on laisse réagir à la température ambiante pendant 6 heures. On évapore le solvant sous vide puis on neutralise la phase aqueuse résiduelle par addition d'acide chlorhydrique concentré jusqu'à obtention d'un pH de 6. On filtre le solide obtenu et on reprend ce dernier dans l'éther isopropylique. On filtre et on sèche le solide au dessiccateur sous vide. La masse récupérée est de 0,05 g, ce qui correspond à un rendement de 30%.

Les analyses en RMN ¹H (200MHz ; DMSO) δ ppm ont donné les résultats suivants :
3.8 (6H ; d), 5.1 (2H ; s), 6.9 (4H ; m), 7.2 (1H ; s), 7.4 (2H ; d), 11.6 (1H ; s), 12.7 (1H ; s).
La structure du composé obtenu est conforme à la structure attendue.

### Exemple n°6 : synthèse de l'acide 5-(4-cyano-benzyloxy)-6-méthoxy-1H-indole-2-carboxylique :

a) Synthèse de l'ester éthylique de l'acide 5-(4-cyano-benzyloxy)-6-méthoxy-1 H-indole-2-carboxylique de formule : 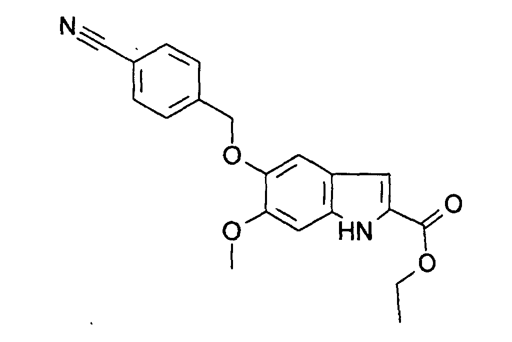
   Dans un réacteur préalablement séché à l'étuve, on introduit 1,6 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1H-indole-2-carboxylique à 0°C, et on laisse réagir 30 minutes à cette température afin de former l'alcoolate. On ajoute ensuite 1,3 équivalents de bromure de 4-cyano benzyle et on laisse réagir jusqu'au retour à la température ambiante, avec agitation pendant 1h30. On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide et on purifie le résidu obtenu sur colonne de silice en utilisant le dichlorométhane comme éluant. La masse récupérée est de 230 mg, ce qui correspond à un rendement de 50%.
b) Synthèse de l'acide 5-(4-cyano-benzyloxy)-6-méthoxy-1H-indole-2-carboxylique de formule : 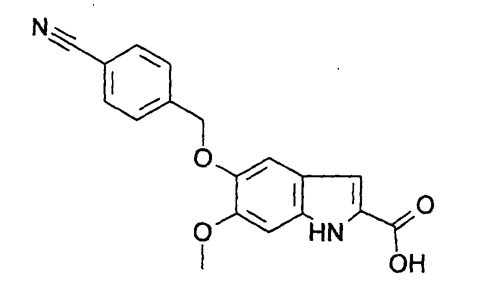
   Dans un réacteur, on solubilise 0,230 g d'ester éthylique de l'acide 5-(4-cyanobenzyloxy)-6-méthoxy-1H-indole-2-carboxylique dans 10 ml d'acétone et 5 ml de méthanol. On ajoute ensuite 10 ml de soude 1N. La réaction est complète en 1h30. On évapore les solvants sous vide et on acidifie la phase aqueuse résiduelle par addition d'acide chlorhydrique concentré jusqu'à obtention d'un pH de 1. On purifie le composé obtenu par solubilisation dans l'éthanol et précipitation dans l'eau sous agitation. La masse récupérée est de 0,05 g, ce qui correspond à un rendement de 25%.

Les analyses en RMN ¹H (200MHz ; DMSO) δ ppm ont donné les résultats suivants :
3.8 (3H ; s), 5.1 (2H ; s), 7 (3H ; t), 7.6 (2H ; d), 7.9 (2H ; d), 11.7 (1H ; s).
La structure du composé obtenu est conforme à la structure attendue.

### Exemple n°7 : synthèse de l'acide 3-H-benzo[E] indole-2-carboxylique :

a) Synthèse de l'ester méthylique de l'acide 2-azido-3-naphtalène-1-yl-acrylique de formule 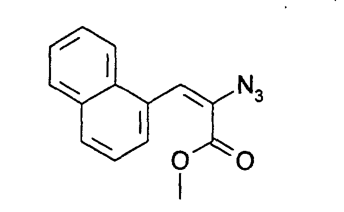
   Dans un réacteur, on introduit 20 ml de méthylate de sodium en solution à 30%. On refroidit à O°C, puis on ajoute lentement 5 g de naphtaldéhyde sous argon. On ajoute ensuite goutte à goutte 8,3 g d'azido acétate d'éthyle, dilués dans 10 ml de méthanol. On laisse réagir 5 heures à O°C. On ajoute 50 ml d'eau à la réaction et on extrait par 2 x 30 ml de dichlorométhane. On sèche la phase organique sur sulfate de sodium et on évapore à sec sous vide et on purifie le produit obtenu sur colonne de silice en utilisant du dichlorométhane comme éluant. La masse récupérée est de 4 g ce qui correspond à un rendement de 70%.
b) Synthèse de l'ester méthylique de l'acide 3-H-benzo [E] indole-2-carboxylique de formule : 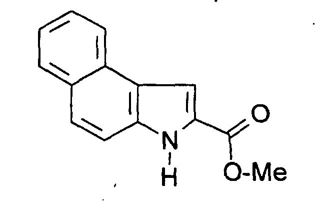
   Dans un réacteur, on solubilise 3 g d'ester méthylique de l'acide 2-azido-3-naphtalène-1-yl-acrylique, dans 20 ml de toluène. On porte le mélange à reflux pendant 3 heures. On évapore le solvant sous vide puis on reprend le résidu dans 20 ml d'heptane chaud. On obtient un solide qui précipite. On filtre et on sèche le solide au dessiccateur sous vide. La masse récupérée est de 3,1 g ce qui correspond à un rendement de 89%.
c) Synthèse de l'acide 3-H-benzo [E] indole-2-carboxylique de formule 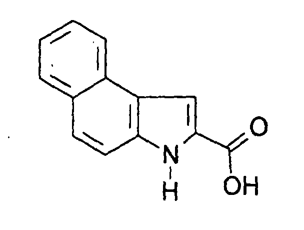
   Dans un réacteur, on solubilise 1 g d'ester méthylique de l'acide 3-H-benzo [E] indole-2-carboxylique, dans 10 ml de méthanol et 20 ml d'acétone. On ajoute ensuite 50 ml de soude 1N. On laisse réagir 1h30 à température ambiante. On évapore les solvants sous vide et on acidifie la phase aqueuse résiduelle en ajoutant de l'acide chlorhydrique concentré jusqu'à obtention d'un pH de 1. On filtre le solide et on le recristallise dans un mélange eau/éthanol. La masse récupérée est de 0,5 g ce qui correspond à un rendement de 53%.

Les analyses en RMN ¹H (200MHz ; DMSO) δ ppm ont donné les résultats suivants :
7.75 (5H ; m), 8.1 (1H ; d), 8.5 (1H; d), 12.3 (1H ; s), 13.1 (1H ; s)

| Analyse Elémentaire : | | | | |
|---|---|---|---|---|
| | C en % | H en % | N en % | O en % |
| théorique | 73.92 | 4.29 | 6,63 | 15.15 |
| trouvée | 73.58 | 4,23 | 6.52 | 15.15 |

### Exemple n°8 : Synthèse de l'acide 3-méthyl-3H-benzo [E] indole-2-carboxylique

a) Synthèse de l'ester méthylique de l'acide 3-méthyl-3-H-benzo [E] indole-2-carboxylique de formule 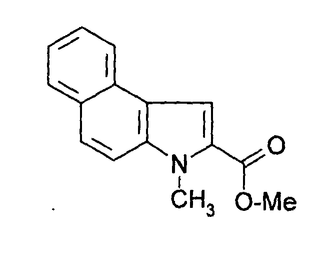
   Dans un réacteur on introduit 1,1 équivalents d'hydrure de sodium (NaH) sous argon et 5 ml de diméthylformamide (DMF) anhydre. On ajoute 0,1 g d'ester méthylique de l'acide 3-H-benzo [E] indole-2-carboxylique et on laisse sous agitation jusqu'à obtention d'un milieu homogène. On ajoute ensuite 0,1 ml d'iodure de méthyle, et on laisse réagir à température ambiante. La réaction est complète en 30 minutes. On ajoute ensuite 30 ml d'une solution saturée en NaCl puis on extrait par deux fois 50 ml de dichlorométhane. On concentre la phase organique à sec sous vide. La masse récupérée est de 0,11 g ce qui correspond à un rendement de 100%.
b) Synthèse de l'acide 3-méthyl-3-H-benzo [E] indole-2-carboxylique de formule : 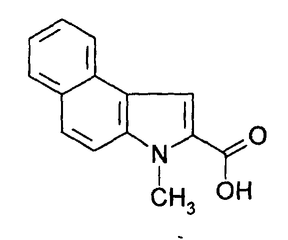
   Dans un réacteur, on solubilise 0,1 g d'ester méthylique de l'acide 3-méthyl-3-H-benzo [E] indole-2-carboxylique dans 7 ml d'acétone. On ajoute ensuite 10 ml de soude 1N. On laisse réagir à température ambiante pendant 1 heure. On évapore le solvant sous vide et on neutralise la phase aqueuse résiduelle en portant le pH à 6 par addition d'acide chlorhydrique concentré. Le solide obtenu est lavé plusieurs fois par de l'eau.
   La masse récupérée est de 0,057 g ce qui correspond à un rendement de 61%.

Les analyses en RMN ¹H (200MHz ; DMSO) δ ppm ont donné les résultats suivants :
7.35 (2H ; m), 7.7 (4H ; m), 8.2 (1H ; d), 12.7 (1H ; s)

### Exemple n°9 : synthèse de l'acide 1-(3,5-bis-trifluorométhyl-benzyl)-5-(3,5-bis-trifluorométhyl-benzyloxy)-6-méthoxy-1H-indole-2-carboxylique :

a) Synthèse de l'ester éthylique de l'acide 1-(3,5-bis-trifluorométhylbenzyl)-5-(3,5-bis-trifluorométhyl-benzyloxy)-6-méthoxy-1H-indole-2-carboxylique de formule : 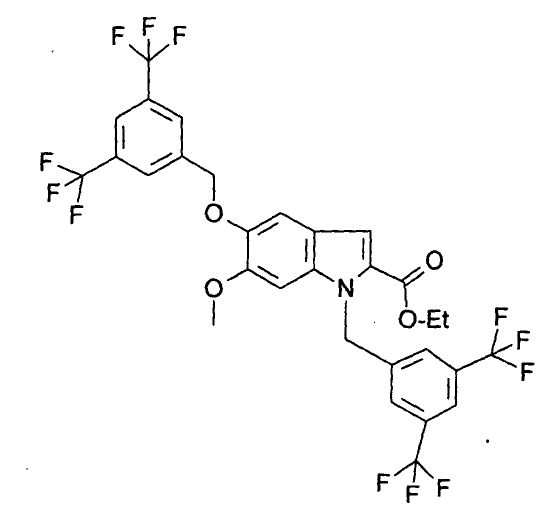
   Dans un réacteur préalablement séché à l'étuve, on introduit 1,6 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1H-indole-2-carboxylique à +3°C, et on laisse réagir 30 minutes à cette température afin de former l'alcoolate. On ajoute ensuite 1,4 équivalents de bromure de 3,5-bis-(trifluorométhyle)-benzyle et on laisse réagir à cette température pendant 1h30. On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide et on purifie le résidu obtenu sur colonne de silice en utilisant le dichlorométhane comme éluant. La masse récupérée est de 200 mg ce qui correspond à un rendement de 40%.
b) Synthèse de l'acide 1-(3,5-bis-trifluorométhyl-benzyl)-5-(3,5-bis-trifluorométhyl-benzyloxy)-6-méthoxy-1H-indole-2-carboxylique de formule : 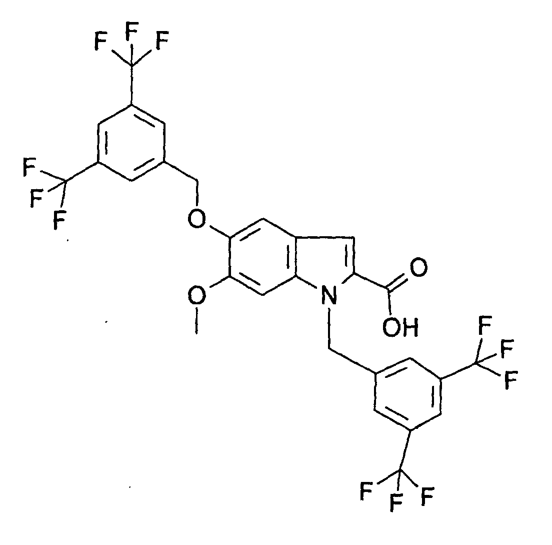
   Dans un réacteur, on solubilise 0,2 g d'ester éthylique de l'acide 1-(3,5-bis-trifluorométhyl-benzyl)-5-(3,5-bis-trifluorométhyl-benzyloxy)-6-méthoxy-1 H-indole-2-carboxylique dans 10 ml d'acétone et 5 ml de méthanol. On ajoute ensuite 10 ml de soude 1N. On laisse réagir 2 heures à température ambiante. On évapore sous vide les solvants et on acidifie la phase aqueuse résiduelle par addition d'acide chlorhydrique concentré jusqu'à obtention d'un pH de 3. On purifie par reprise dans l'éther isopropylique. La masse récupérée est de 0,08 g ce qui correspond à un rendement de 45%.

Les analyses en RMN ¹H (200MHz ; DMSO) δ ppm ont donné les résultats suivants :
3.8 (3H ; s), 5.3 (2H ; s), 6.2 (2H ; s), 7.2 (3H ; t), 8.0 (6H ; m)
La structure du composé obtenu est conforme à la structure attendue.

### Exemple n°10 : évaluation de l'effet inhibiteur de composés de l'invention sur les 5α-réductases de types 1 et 2 :

Les séquences codantes (Acides désoxyribonlucléiques complémentaires : cDNA) de la 5α-réductase 1 et de la 5α-réductase 2 ont été clonées dans le vecteur d'expression eucaryote pSG5 (Stratagene). Les enzymes ont été surexprimées après transfection transitoire de cellules COS7 (ATCC, CRL1651).

Les cDNAs de la 5α-réductase 1 et de la 5α-réductase 2 ont été obtenus par transcription inverse et réaction de polymérisation en chaîne (Polymerase chain reaction : PCR) à l'aide d'amorces spécifiques à partir d'ARN total de testicules humains (vendu par la société Clontech).
Les amorces utilisées pour obtenir le cDNA de la 5α-réductase 1 sont :

Brin + : 5' CCCAGCCCTGGCGATGGCAAC 3',

Brin - : 5' GGATATTCAACCTCCATTTCAG 3'.

Les amorces utilisées pour obtenir le cDNA de la 5α-réductase 2 sont :

Brin + : 5' GCGATGCAGGTTCAGTG 3',

Brin - : 5' ATTGTGGGAGCTCTGCT 3'.

Par des techniques classiques de génie génétique, le cDNA de la 5α-réductase 1 obtenu a été inséré au site BamHI de pSG5 et celui de la 5α-réductase 2 au site EcoRI (voir Magnatis et col., Molecular Cloning, Cold Spring Harber, 1989)

Les clones positifs (recombinants) ont été identifiés par la technique d'hybridation avec une sonde froide (Plex luminescent kits, Millipore) et cartographiés par digestions enzymatiques et séquençage partiel.
Après transfection transitoire les cellules COS7 sont lysées dans un tampon Tris-HCI 10 mM, pH=7 / 150 mM KCl / 1 mM EDTA par 3 cycles de congélation/décongélation. L'homogénat est centrifugé à 100 000 g pendant 1 heure. Les culots contenant la 5α-réductase 1 ou la 5α-réductase 2 sont repris dans un tampon phosphate 40 mM, pH 6.5 ou citrate 40 mM, pH 5.5 respectivement pour la 5α-réductase 1 ou 5α-réductase 2.
5 µg de protéines ainsi obtenues sont incubés dans un puits d'une plaque 96 puits (NUNC) en présence de 1 nM de ¹⁴C-Testostérone (Amersham) et de 5 mM de Nicotinamide Adenosine Dinucleotide Phosphate, forme réduite (NADPH) (Sigma) dans le tampon correspondant (tampon phosphate 40 mM, pH 6.5 ou citrate 40 mM, pH 5.5 respectivement pour la 5α-réductase 1 ou 5α-réductase 2) pendant 50 minutes à 37°C après addition des produits à tester.
Les produits à tester sont ajoutés aux concentrations de 10⁻⁴ M à 10⁻¹⁰M, dilués dans du tampon phosphate 40 mM, pH 6.5 ou citrate 40 mM, pH 5.5 respectivement pour la 5α-réductase 1 ou 5α-réductase 2.
Les mélanges réactionnels sont ensuite déposés directement sur un plaque de silice (HPTLC, 60F 254, Merck) et soumis à une chromatographie (solvant = 10% diéthyléther, 90% dichlorométhane). Ils sont ensuite analysés par autoradiographie digitale (Digital Autoradiography, Berthold).
On mesure l'inhibition de l'activité des isoenzymes en calculant le pourcentage de dihydrotestostérone formée à partir de ¹⁴C-testostérone par rapport à un témoin non traité.
Les résultats sont présentés dans le tableau suivant dans lequel seuls les composés 1, 2, 7 et 8 sont inclus dans les composés de formula (I):

| | | | | | | 5α-réductases : IC50 | |
|---|---|---|---|---|---|---|---|
| ex | X | Y | Z | R₁ | R₂ | Type I (µM) | Type II (nM) |
| 1 | OCH₂Ph | H | - OMe | H | H | 1-4 | >100 000 |
| 2 | OCH₂Ph(CF₃)₂ | H | -OMe | H | H | 10 | >100 000 |
| 3 | H | OCH₂Ph(CF₃)₂ | -OMe | H | H | 0.5 | 1-5 |
| 4 | H | OCH₂Ph(OMe)₃ | -OMe | H | H | >100 | 10 |
| 5 | H | OCH₂Ph (OMe) | -OMe | H | H | 1-5 | 10 |
| 6 | H | OCH₂Ph(CN) | -OMe | H | H | 5 | 40 |
| 7 | Ph | | H | H | H | 3 | 6000 |
| 8 | Ph | | H | H | Me | 0,5 | 10000 |
| a | H | OCH₂Ph | -OMe | H | H | 1 | 1 |
| b | H | OCH₂Ph | -OCH₂Ph | Me | H | >100 | 1000 |
| c | H | OCH₂Ph | -OCH₂Ph | H | H | >100 | 5 |
| Ph = -C₆H₅ ; Me = -CH₃ | | | | | | | |
| a, b, c : composés dont la synthèse n'est pas exemplifiée. | | | | | | | |

Les composés des exemples 1 et 2 présentent une spécificité très marquée vis-à-vis de la 5α-réductase de type 1.

Les composés des exemples 7 et 8 présentent une spécificité marquée vis-à-vis de la 5α-réductase de type 1.

Les composés des exemples 3, 5, 6 et le composé a sont de très bons inhibiteurs des deux types de 5α-réductase.

Le composé de l'exemple 4 et le composé c présentent une spécificité plus marquée pour la 5α-réductase de type 2.

Le composé b est un inhibiteur moyen avec une spécificité plus marquée pour le type I.

### Exemple n°10 : Exemples de compositions contenant un dérivé indole-carboxylique.

Ces compositions sont obtenues par les techniques habituelles couramment utilisées en cosmétique ou en pharmacie.

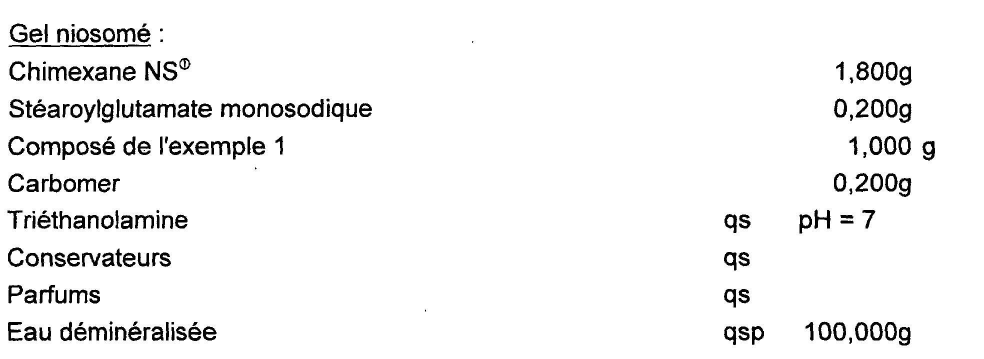

On applique ce gel sur le cuir chevelu, une à deux fois par jour.

| Lotion antichute : | |
|---|---|
| Composé de l'exemple 3 | 2,000 g |
| Propylène glycol | 30,000 g |
| Alcool éthylique | 40,500 g |
| Eau | qsp 100,000 g |

On applique cette lotion sur le cuir chevelu, une à deux fois par jour, à raison d'1 ml par application.

| Lotion antichute épaissie : | |
|---|---|
| Composé de l'exemple 7 | 5,000 g |
| Kawaïne | 2,000 g |
| Hydroxypropylcellulose (Klucel G de la société Hercules) | 3,500 g |
| Alcool éthylique | qsp 100,000 g |

On applique cette lotion épaissie sur le cuir chevelu, une à deux fois par jour, à raison d'1 ml par application.

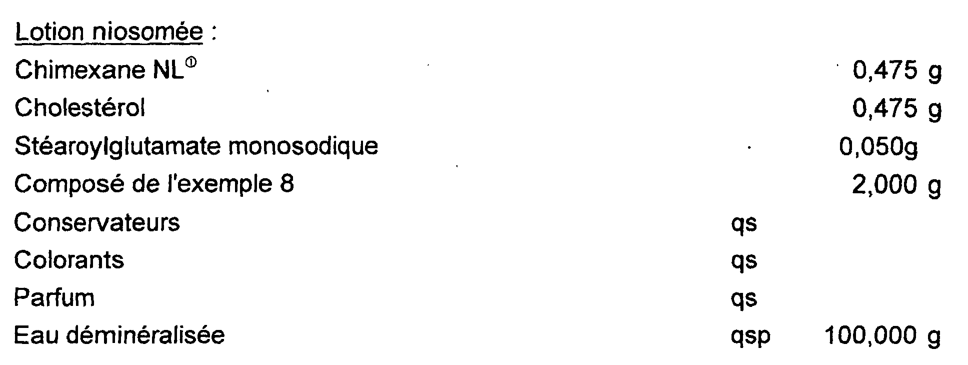

On applique cette lotion sur le cuir chevelu, une à deux fois par jour, à raison d'1 ml par application.

| lotion antichute : | |
|---|---|
| Composé de l'exemple 3 | 2,500 g |
| Monométhyléther de propylèneglycol (Dowanol PM de Dow Chemical) | 20,000 g |
| Hydroxy Alcool éthylique | 40,000g |
| Minoxidil | 2,000 g |
| Eau | qsp 100,000 g |

On applique cette lotion épaissie sur le cuir chevelu, une à deux fois par jour, à raison d'1 ml par application.

| Lotion antichute : | |
|---|---|
| Composé de l'exemple 7 | 0,200 g |
| Propylène glycol | 10,000g |
| Alcool isopropylique | qsp 100,000g |

On applique 1 ml de cette lotion sur le cuir chevelu, à la fréquence de une à deux fois par jour.

Avec chacune des compositions décrites dans les exemples ci-dessus, on a constaté, après plusieurs mois de traitement et selon les sujets traités, un ralentissement de la chute des cheveux et/ou un effet repousse.

## Revendications

1. Utilisation d'une quantité efficace d'au moins un composé répondant à la formule générale (I) 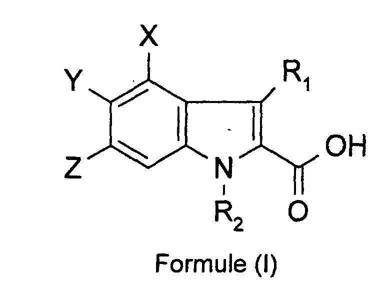 dans laquelle :
X représente un radical -O-CHR₃R₄, dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Y représente un atome d'hydrogène ou un radical -O-CHR₃R₄, dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Z représente un radical -O-R₅ dans lequel R₅ est un radical alkyle en C₁-C₄ choisi parmi les radicaux méthyle, éthyle, propyle, isopropyle, butyle, isobutyle ou tertiobutyle;
ou X et Y forment pris ensemble, avec les 2 atomes de carbone les portant, un cycle ou un hétérocycle ayant 5 ou 6 atomes de carbone et Z représente un atome d'hydrogène ou un radical -O-R₅ dans lequel R₅ est un radical alkyle en C₁-C₄ choisi parmi les radicaux méthyle, éthyle, propyle, isopropyle, butyle, isobutyle ou tertiobutyle;
R₁ représente un atome d'hydrogène ou un radical alkyle en C₁ - C₆ ou un radical aralkyle en C₆ - C₁₂, éventuellement substitué ;
R₂ représente un atome d'hydrogène ou un radical alkyle en C₁ - C₆ ou un radical -CHR₃R₄, dans lequel R₃ et R₄ ont les définitions précédentes ;
étant entendu que lorsque R₃ ou R₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène,
pour la fabrication d'une composition destinée au traitement des désordres liés à une suractivité de la 5α-réductase de type 1, lesdits désordres sont choisis parmi l'acné, l'hirsutisme, la séborrhée, l'alopécie androgénique, les chéloïdes et les adhésions, les carcinomes prostatiques, l'hyperplasie bénigne de la prostate, le syndrome polycystique ovarien, le syndrome prémenstruel, le cancer du poumon chez l'homme, la puberté précoce, la maladie de Fox et Fordyce.

2. Utilisation selon la revendication précédente, **caractérisée par le fait que** le composé ou la composition sont destinés à induire et/ou stimuler la croissance des cheveux et/ou freiner leur chute et/ou à traiter l'hyperséborrhée et/ou l'acné.

3. Utilisation selon l'une quelconque des revendications 1 et 2, **caractérisée par le fait que** le composé est utilisé en une quantité représentant de 0,001% à 10% du poids total de la composition.

4. Utilisation selon la revendication 3, **caractérisée par le fait que** le composé est utilisé en une quantité représentant de 0,01% à 5% du poids total de la composition.

5. Procédé de traitement cosmétique de la peau, des cheveux et/ou du cuir chevelu, **caractérisé par le fait qu'**il consiste à appliquer sur les cheveux et/ou le cuir chevelu et/ou sur la peau, une composition cosmétique comprenant un composé de formule (I') tel que définie dans l'une quelconque des revendications 12 à 14, à laisser celle-ci au contact de la peau, des cheveux et/ou du cuir chevelu, et éventuellement à rincer.

6. Dérivé indole-carboxylique répondant à la formule générale (I') : 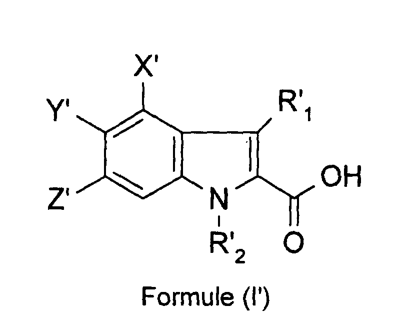 dans laquelle :
X' représente un radical -O-CHR'₃R'₄, dans lequel R'₃ est un atome d'hydrogène ou un radical phényle éventuellement substitué ou encore un hétérocycle ayant 5 ou 6 chaînons et R'₄ est un radical phényle éventuellement substitué ou encore un hétérocycle ayant 5 ou 6 chaînons, ou R'₃ et R'₄ pris ensemble forment avec l'atome de carbone un cycle ou un hétérocycle ayant 5 ou 6 chaînons ;
Y' représente un atome d'hydrogène ou un radical -O-CHR₃R₄, dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Z' représente un radical -O-R'₅ dans lequel R'₅ est un radical alkyle en C₁-C₄ choisi parmi les radicaux méthyle, éthyle, propyle, isopropyle, butyle, isobutyle ou tertiobutyle;
ou X' et Y' forment pris ensemble, avec les 2 atomes de carbone les portant, un cycle ou un hétérocycle ayant 5 ou 6 atomes de carbone et Z représente un atome d'hydrogène ou un radical -O-R'₅ dans lequel R'₅ est un radical alkyle en C₁-C₄ choisi parmi les radicaux méthyle, éthyle, propyle, isopropyle, butyle, isobutyle ou tertiobutyle;
R'₁ représente un atome d'hydrogène ou un radical alkyle en C₁ - C₆ ou un radical aralkyle en C₆ - C₁₂, éventuellement substitué ;
R'₂ représente un atome d'hydrogène ou un radical alkyle en C₁ - C₆ ou un radical -CHR'₃R'₄, dans lequel R'₃ et R'₄ ont les définitions précédentes ;
ou ses esters ou ses isomères optiques, seuls ou en mélange en toutes proportions, ou encore ses sels pharmaceutiquement acceptables.
étant entendu :
que lorsque R'₁, R'₂ et Z' représentent un atome d'hydrogène, alors X' et Y' ne doivent pas former pris ensemble, avec les 2 atomes de carbone les portant, un cycle ayant 5 à 7 atomes de carbone,
que lorsque R'₂ et Z' représentent un atome d'hydrogène et R'₁ un radical CH₃ alors X' et Y' ne doivent pas former pris ensemble, avec les 2 atomes de carbone les portant, un cycle ayant 6 atomes de carbone,
que lorsque X' est un radical -O-CH₂Phényl et R'₁, R'₂ et Y' représentent un atome d'hydrogène alors Z' ne doit pas être un radical OCH₃,
que le dérivé de formule (I') n'est pas l'acide 3H-pyrrolo-[3,2-f]-quinoline-carboxylique ou l'acide 2,3-dihydro-7H-1,4-dioxino'2,3-elindole-8-carboxylique,
et que lorsque R'₃ ou R'₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène.

7. Dérivé selon la revendication 6, **caractérisé par le fait que** R'₁ est un radical alkyle en C₁ - C₆ éventuellement substitué.

8. Dérivé selon l'une quelconque des revendications 6 ou 7, **caractérisé par le fait que** R'₁ est un radical aralkyle en C₆ - C₁₂ éventuellement substitué.

9. Dérivé selon l'une quelconque des revendications 6 à 8, **caractérisé par le fait que** R'₂ est un radical alkyle en C₁ - C₆.

10. Dérivé selon l'une quelconque des revendications 6 à 9, **caractérisé par le fait que** quand R'₃ ou R'₄ indépendamment l'un de l'autre sont un hétérocycle celui-ci est choisi parmi la pyridine, la quinoline, l'imidazole, le benzimidazole, le tétrahydrofuranne ou le furanne.

11. Dérivé selon l'une quelconque des revendications 6 à 10, **caractérisé par le fait qu'**il est choisi parmi :
l'acide 4-(3,5-bis-trifluorométhyl-benzyloxy)-6-méthoxy-1 H-indole-2-carboxylique,
l'acide 3-méthyl-3H-benzo [E] indole-2-carboxylique,
l'acide 3-benzyl-3H-benzo [E] indole-2-carboxylique,
l'acide 3-(3,5-bis-trifluorométhyl-benzyl)-3H-benzo [E] indole-2-carboxylique.

12. Composition comprenant au moins un composé de formule (I') tel que défini selon l'une quelconque des revendications 6 à 11.

13. Composition selon la revendication 12, **caractérisée par le fait qu'**elle est destinée à un usage cosmétique ou pharmaceutique.

14. Composition selon l'une quelconque des revendications 11 à 12, **caractérisée par le fait qu'**elle comprend au moins un composé de formule (l') en une quantité représentant de 0,001% à 10% du poids total de la composition et préférentiellement en une quantité représentant de 0,01% à 2% du poids total de la composition.

15. Composition selon l'une quelconque des revendications 12 à 14, **caractérisée par le fait qu'**elle comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

16. Composition selon l'une quelconque des revendications 12 à 15, **caractérisée par le fait qu'**elle est destinée à traiter les désordres liés à une suractivité de la 5α-réductase de type 1, lesdits désordres sont choisis parmi la séborrhée et/ou l'acné et/ou l'hirsutisme et/ou l'alopécie androgénétique.

17. A titre de médicaments les composés de formule générale (I') 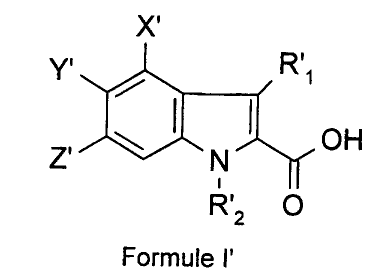 tels que définis dans les revendications 6 à 11.

## Claims

1. Use of an effective amount of at least one compound corresponding to the general formula (I) 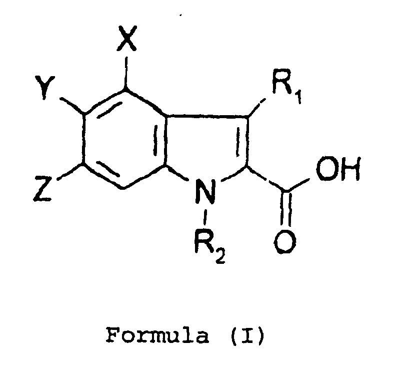 in which:
X represents a radical -O-CHR₃R₄, in which R₃ is a hydrogen atom or an optionally substituted phenyl radical or alternatively a 5- or 6-membered heterocycle and R₄ is an optionally substituted phenyl radical or alternatively a 5- or 6-membered heterocycle, or R₃ and R₄ taken together form, with the carbon atom, a 5- or 6-membered ring or heterocycle;
Y represents a hydrogen atom or a radical -O-CHR₃R₄, in which R₃ is a hydrogen atom or an optionally substituted phenyl radical or alternatively a 5- or 6-membered heterocycle and R₄ is an optionally substituted phenyl radical or alternatively a 5- or 6-membered heterocycle, or R₃ and R₄ taken together form, with the carbon atom, a 5- or 6-membered ring or heterocycle;
Z represents a radical -O-R₅ in which R₅ is a C₁-C₄ alkyl radical chosen from methyl, ethyl, propyl, isopropyl, butyl, isobutyl and tert-butyl radicals;
or X and Y taken together form, with the two carbon atoms bearing them, a ring or heterocycle containing 5 or 6 carbon atoms and Z represents a hydrogen atom or a radical -O-R₅ in which R₅ is a C₁-C₄ alkyl radical chosen from methyl, ethyl, propyl, isopropyl, butyl, isobutyl and tert-butyl radicals;
R₁ represents a hydrogen atom or an optionally substituted C₁-C₆ alkyl or C₆-C₁₂ aralkyl radical;
R₂ represents a hydrogen atom, a C₁-C₆ alkyl radical or a radical -CHR₃R₄, in which R₃ and R₄ have the above definitions;
it being understood that when R₃ or R₄, independently of each other, is a substituted phenyl radical, the said radical is substituted with a cyano group (-CN), a trifluoromethyl group (-CF₃), a methoxy radical (-O-CH₃) or a halogen atom,
for the manufacture of a composition intended for the treatment of disorders associated with overactivity of type 1 5α-reductase, the said disorders being selected from acne, hirsutism, seborrhoea, androgenic alopecia, cheloids and adhesions, prostate carcinomas, benign hyperplasia of the prostate, ovarian polycystic syndrome, premenstrual syndrome, lung cancer in man, precocious puberty and Fox-Fordyce disease.

2. Use according to the preceding claim, **characterized in that** the compound or the composition is intended to induce and/or stimulate hair growth and/or to slow down hair loss and/or to treat hyperseborrhoea and/or acne.

3. Use according to any one of Claims 1 to 2, **characterized in that** the compound is used in an amount representing from 0.001% to 10% of the total weight of the composition.

4. Use according to Claim 3, **characterized in that** the compound is used in an amount representing from 0.01% to 5% of the total weight of the composition.

5. Cosmetic treatment process for the skin and/or the hair and/or the scalp, **characterized in that** it consists in applying to the hair and/or the scalp and/or the skin a cosmetic composition comprising a compound of formula (I') as defined in any one of Claims 12 to 14, in leaving this composition in contact with the skin and/or the hair and/or the scalp, and optionally in rinsing it out.

6. Indolecarboxylic derivative corresponding to the general formula (I'): 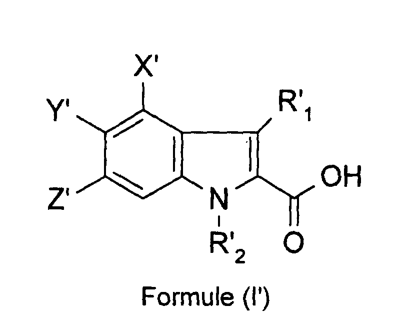 in which:
X' represents a radical -O-CHR'₃R'₄, in which R'₃ is a hydrogen atom or an optionally substituted phenyl radical or alternatively a 5- or 6-membered heterocycle and R'₄ is an optionally substituted phenyl radical or alternatively a 5- or 6-membered heterocycle, or R'₃ and R'₄ taken together form, with the carbon atom, a 5- or 6-membered ring or heterocycle;
Y' represents a hydrogen atom or a radical -O-CHR₃R₄, in which R₃ is a hydrogen atom or an optionally substituted phenyl radical or alternatively a 5- or 6-membered heterocycle and R₄ is an optionally substituted phenyl radical or alternatively a 5- or 6-membered heterocycle, or R₃ and R₄ taken together form, with the carbon atom, a 5- or 6-membered ring or heterocycle;
Z' represents a radical -O-R'₅ in which R'₅ is a C₁-C₄ alkyl radical chosen from methyl, ethyl, propyl, isopropyl, butyl, isobutyl and tert-butyl radicals;
or X' and Y' taken together form, with the two carbon atoms bearing them, a ring or heterocycle containing 5 or 6 carbon atoms and Z represents a hydrogen atom or a radical -O-R'₅ in which R'₅ is a C₁-C₄ alkyl radical chosen from methyl, ethyl, propyl, isopropyl, butyl, isobutyl and tert-butyl radicals;
R'₁ represents a hydrogen atom or an optionally substituted C₁-C₆ alkyl or C₆-C₁₂ aralkyl radical;
R'₂ represents a hydrogen atom, a C₁-C₆ alkyl radical or a radical -CHR'₃R'₄, in which R'₃ and R'₄ have the above definitions;
or the esters or optical isomers thereof, alone or as a mixture in all proportions, or the pharmaceutically acceptable salts thereof,
it being understood that:
when R'₁, R'₂ and Z' represent a hydrogen atom, then X' and Y' must not form together, with the two carbon atoms bearing them, a ring containing 5 to 7 carbon atoms,
when R'₂ and Z represent a hydrogen atom and R'₁ represents a CH₃ radical, then X' and Y' must not form together, with the two carbon atoms bearing them, a ring containing 6 carbon atoms,
when X' is an -O-CH₂Phenyl radical and R'₁, R'₂ and Y' represent a hydrogen atom, then Z' must not be an OCH₃ radical,
the derivative of formula (I') is not 3H-pyrrolo[3,2-f]quinolinecarboxylic acid or 2,3-dihydro-7H-1,4-dioxino-[2,3-e] indole-8-carboxylic acid, and
when R'₃ or R'₄, independently of each other, is a substituted phenyl radical, the said radical is substituted with a cyano group (-CN), a trifluoromethyl group (-CF₃), a methoxy radical (-O-CH₃) or a halogen atom.

7. Derivative according to Claim 6, **characterized in that** R'₁ is an optionally substituted C₁-C₆ alkyl radical.

8. Derivative according to either of Claims 6 and 7, **characterized in that** R'₁ is an optionally substituted C₆-C₁₂ aralkyl radical.

9. Derivative according to any one of Claims 6 to 8, **characterized in that** R'₂ is a C₁-C₆ alkyl radical.

10. Derivative according to any one of Claims 6 to 9, **characterized in that** when R'₃ or R'₄, independently of each other, is a heterocycle, this heterocycle is chosen from pyridine, quinoline, imidazole, benzimidazole, tetrahydrofuran and furan.

11. Derivative according to any one of Claims 6 to 10, **characterized in that** it is chosen from:
4-[3,5-bis(trifluoromethyl)benzyloxy]-6-methoxy-1H-indole-2-carboxylic acid,
3-methyl-3H-benz[e]indole-2-carboxylic acid,
3-benzyl-3H-benz[e]indole-2-carboxylic acid,
3-[3,5-bis(trifluoromethyl)benzyl]-3H-benz[e]indole-2-carboxylic acid.

12. Composition comprising at least one compound of formula (I') as defined according to any one of Claims 6 to 11.

13. Composition according to Claim 12, **characterized in that** it is intended for cosmetic or pharmaceutical use.

14. Composition according to any one of Claims 11 to 12, **characterized in that** it comprises at least one compound of formula (I') in an amount representing from 0.001% to 10% of the total weight of the composition, and preferably in an amount representing from 0.01% to 2% of the total weight of the composition.

15. Composition according to any one of Claims 12 to 14, **characterized in that** it also comprises at least one agent chosen from antibacterial agents, antiparasitic agents, antifungal agents, antiviral agents, anti-inflammatory agents, anti-pruriginous agents, anaesthetics, keratolytic agents, anti-free-radical agents, anti-seborrhoeic agents, antidandruff agents, anti-acne agents and/or agents for reducing skin differentiation and/or proliferation and/or pigmentation, and extracts of plant or bacterial origin.

16. Composition according to any one of Claims 12 to 15, **characterized in that** it is intended to treat disorders associated with overactivity of type 1 5α-reductase, the said disorders being selected from seborrhoea and/or acne and/or hirsutism and/or androgenic alopecia.

17. As medicinal compounds, the compounds of general formula (I') 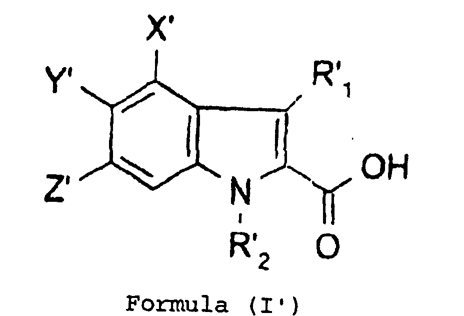 as defined in Claims 6 to 11.

## Patentansprüche

1. Verwendung einer wirksamen Menge mindestens einer Verbindung der allgemeinen Formel (I) 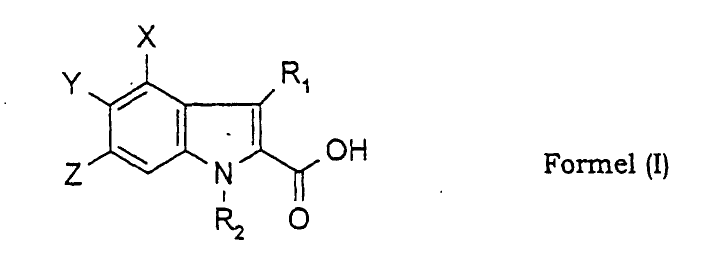 worin bedeuten:
X eine Gruppe -O-CHR₃R₄, wobei R₃ ein Wasserstoffatom oder eine gegebenenfalls substituierte Phenylgruppe oder einen 5-oder 6-gliedrigen Heterocyclus und R₄ eine gegebenenfalls substituierte Phenylgruppe oder einen 5- oder 6-gliedrigen Heterocyclus bedeuten oder wobei R₃ und R₄ gemeinsam mit dem Kohlenstoffatom einen 5- oder 6-gliedrigen Ring oder Heterocyclus bilden;
Y ein Wasserstoffatom oder eine Gruppe -O-CHR₃R₄, wobei R₃ ein Wasserstoffatom oder eine gegebenenfalls substituierte Phenylgruppe oder einen 5- oder 6-gliedrigen Heterocyclus und R₄ eine gegebenenfalls substituierte Phenylgruppe oder einen 5-oder 6-gliedrigen Heterocyclus bedeuten oder wobei R₃ und R₄ gerneinsam mit dem Kohlenstoffatom einen 5- oder 6-gliedrigen Ring oder Heterocyclus bilden;
Z eine Gruppe -O-R₅, wobei R₅ eine C₁₋₄-Alkylgruppe bedeutet, die unter Methyl, Ethyl, Propyl, Isopropyl, Butyl, Isobutyl oder *t*-Butyl ausgewählt ist;
oder X und Y bilden gemeinsam mit den beiden Kohlenstoffatomen, die sie tragen, einen Ring oder Heterocyclus mit 5 oder 6 Kohlenstoffatomen und Z bedeutet Wasserstoff oder eine Gruppe -O-R₅, wobei R₅ eine C₁₋₄-Alkylgruppe bedeutet, die unter Methyl, Ethyl, Propyl, Isopropyl, Butyl, Isobutyl oder *t*-Butyl ausgewählt ist;
R₁ ein Wasserstoffatom, eine C₁₋₆-Alkylgruppe oder eine gegebenenfalls substituierte C₆₋₁₂-Aralkylgruppe;
R₂ ein Wasserstoffatom, eine C₁₋₆-Alkylgruppe oder eine Gruppe -CHR₃R₄, wobei R₃ und R₄ die oben angegebenen Bedeutungen aufweisen; mit der Maßgabe, daß, wenn R₃ oder R₄ unabhängig voneinander eine substituierte Phenylgruppe bedeuten, die Phenylgruppe mit einer Cyanogruppe (-CN), Trifluormethylgruppe (-CF₃), Methoxygruppe (-O-CH₃) oder einem Halogenatom substituiert ist,
zur Herstellung einer Zusammensetzung, die zur Behandlung von Störungen vorgesehen ist, die mit einer Überaktivität der 5α-Reduktase vom Typ 1 zusammenhängen, wobei diese Störungen unter Akne, Hirsutismus, Seborrhoe, androgenetischer Alopezie, Keloiden und Verklebungen, Prostatacarzinomen, benigner Prostatahyperplasie, dem Syndrom der polyzystischen Ovarien, dem prämenstruellen Syndrom, Lungenkrebs beim Mann, Pubertas praecox und der Fox-Fordyce-Krankheit ausgewählt sind.

2. Verwendung nach dem vorhergehenden Anspruch, **dadurch gekennzeichnet, daß** die Verbindung oder die Zusammensetzung dazu vorgesehen ist, den Haarwuchs zu induzieren und/oder zu stimulieren und/oder den Haarausfall zu verlangsamen und/oder Hyperseborrhoe und/oder Akne zu behandeln.

3. Verwendung nach einem der Ansprüche 1 und 2, **dadurch gekennzeichnet, daß** die Verbindung in einer Menge von 0,001 bis 10 % des Gesamtgewichts der Zusammensetzung verwendet wird.

4. Verwendung nach Anspruch 3, **dadurch gekennzeichnet, daß** die Verbindung in einer Menge von 0,01 bis 5 % des Gesamtgewichts der Zusammensetzung verwendet wird.

5. Verfahren zur kosmetischen Behandlung der Haut, der Haare und/oder der Kopfhaut, **dadurch gekennzeichnet, daß** es darin besteht, auf die Haare und/oder die Kopfhaut und/oder die Haut eine kosmetische Zusammensetzung aufzutragen, die eine Verbindung der Formel (I') nach einem der Ansprüche 12 bis 14 enthält, diese im Kontakt mit der Haut, den Haaren und/oder der Kopfhaut zu belassen und gegebenenfalls zu spülen.

6. Carboxyindolderivat der allgemeinen Formel (I'): 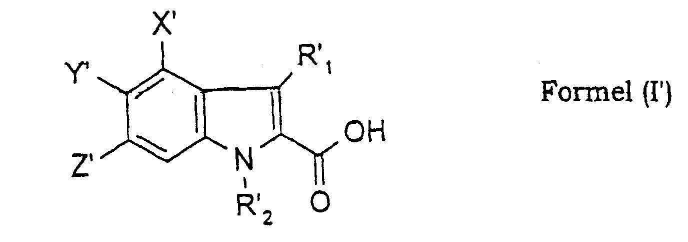 worin bedeuten:
X' eine Gruppe -O-CHR'₃R'₄, wobei R'₃ ein Wasserstoffatom oder eine gegebenenfalls substituierte Phenylgruppe oder einen 5-oder 6-gliedrigen Heterocyclus und R'₄ eine gegebenenfalls substituierte Phenylgruppe oder einen 5- oder 6-gliedrigen Heterocyclus bedeuten oder wobei R'₃ und R'₄ gemeinsam mit dem Kohlenstoffatom einen 5- oder 6-gliedrigen Ring oder Heterocyclus bilden;
Y' ein Wasserstoffatom oder eine Gruppe -O-CHR'₃R'₄, wobei R₃ ein Wasserstoffatom oder eine gegebenenfalls substituierte Phenylgruppe oder einen 5- oder 6-gliedrigen Heterocyclus und R'₄ eine gegebenenfalls substituierte Phenylgruppe oder einen 5-oder 6-gliedrigen Heterocyclus bedeuten oder wobei R'₃ und R'₄ gemeinsam mit dem Kohlenstoffatom einen 5- oder 6-gliedrigen Ring oder Heterocyclus bilden;
Z' eine Gruppe -O-R'₅, wobei R'₅ eine C₁₋₄-Alkylgruppe bedeutet, die unter Methyl, Ethyl, Propyl, Isopropyl, Butyl, Isobutyl oder *t*-Butyl ausgewählt ist;
oder X' und Y' bilden gemeinsam mit den beiden Kohlenstoffatomen, die sie tragen, einen Ring oder Heterocyclus mit 5 oder 6 Kohlenstoffatomen und Z bedeutet Wasserstoff oder eine Gruppe -O-R'₅, wobei R'₅ eine C₁₋₄-Alkylgruppe bedeutet, die unter Methyl, Ethyl, Propyl, Isopropyl, Butyl, Isobutyl oder *t*-Butyl ausgewählt ist;
R'₁ ein Wasserstoffatom, eine C₁₋₆-Alkylgruppe oder eine gegebenenfalls substituierte C₆₋₁₂-Aralkylgruppe;
R'₂ ein Wasserstoffatom, eine C₁₋₆-Alkylgruppe oder eine Gruppe -CHR'₃R'₄, wobei R'₃ und R'₄ die oben angegebenen Bedeutungen aufweisen,
oder die Ester oder optischen Isomere einzeln oder im Gemisch in beliebigen Mengenanteilen oder pharmazeutisch akzeptablen Salze, mit der Maßgabe, daß:
X' und Y' mit den beiden Kohlenstoffatomen, die sie tragen, keinen Ring mit 5 bis 7 Kohlenstoffatomen bilden dürfen, wenn R'₁, R'₂ und Z' ein Wasserstoffatom bedeuten;
X' und Y' nicht gemeinsam mit den beiden Kohlenstoffatomen, die sie tragen, einen Ring mit 6 Kohlenstoffatomen bilden dürfen, wenn R'₂ und Z' ein Wasserstoffatom bedeuten und R'₁ die Gruppe CH₃ ist;
Z' keine Gruppe -O-CH₃ bedeuten darf, wenn X' die Gruppe -O-CH₂-Phenyl ist und R'₁, R'₂ und Y' ein Wasserstoffatom bedeuten;
das Derivat der Formel (I') nicht die 3H-Pyrrolo-[3,2-f]-chinolincarbonsäure oder die 2,3-Dihydro-7H-1,4-dioxino[2,3-e]indolin-8-carbonsäure ist; und
wenn R'₃ oder R'₄ unabhängig voneinander eine gegebenenfalls substituierte Phenylgruppe bedeuten, diese mit einer Cyanogruppe (-CN), Trifluormethylgruppe (-CF₃), Methoxygruppe (-O-CH₃) oder einem Halogen substituiert ist.

7. Derivat nach Anspruch 6, **dadurch gekennzeichnet, daß** R'₁ eine gegebenenfalls substituierte C₁₋₆-Alkylgruppe ist.

8. Derivat nach einem der Ansprüche 6 oder 7, **dadurch gekennzeichnet, daß** R'₁ eine gegebenenfalls substituierte C₆₋₁₂-Aralkylgruppe ist.

9. Derivat nach einem der Ansprüche 6 bis 8, **dadurch gekennzeichnet, daß** R'₂ eine C₁₋₆-Alkylgruppe ist.

10. Derivat nach einem der Ansprüche 6 bis 9, **dadurch gekennzeichnet, daß**, wenn R'₃ oder R'₄ unabhängig voneinander einen Heterocyclus bedeuten, dieser unter Pyridin, Chinolin, Imidazol, Benzimidazol, Tetrahydrofuran oder Furan ausgewählt ist.

11. Derivat nach einem der Ansprüche 6 bis 10, **dadurch gekennzeichnet, daß** es ausgewählt ist unter:
4-(3,5-Bis-trifluormethyl-benzyloxy)-6-methoxy-1H-indol-2-carbonsäure,
3-Methyl-3H-benzo[E]indol-2-carbonsäure,
3-Benzyl-3H-benzo[E]indol-2-carbonsäure und
3-(3,5-Bis-trifluormethyl-benzyl)-3H-benzo[E]indol-2-carbonsäure.

12. Zusammensetzung, die mindestens eine Verbindung der Formel (I') nach einem der Ansprüche 6 bis 11 enthält.

13. Zusammensetzung nach Anspruch 12, **dadurch gekennzeichnet, daß** sie für eine kosmetische oder pharmazeutische Anwendung vorgesehen ist.

14. Zusammensetzung nach einem der Ansprüche 11 bis 12, **dadurch gekennzeichnet, daß** sie mindestens eine Verbindung der Formel (I') in einer Menge von 0,001 bis 10 % des Gesamtgewichts der Zusammensetzung und vorzugsweise in einer Menge von 0,01 bis 2 % des Gesamtgewichts der Zusammensetzung enthält.

15. Zusammensetzung nach einem der Ansprüche 12 bis 14, **dadurch gekennzeichnet, daß** sie auch mindestens einen Wirkstoff enthält, der unter den antibakteriellen Wirkstoffen, antiparasitären Wirkstoffen, antimykotischen Wirkstoffen, antiviralen Wirkstoffen, enzündungshemmenden Wirkstoffen, juckreizstillenden Wirkstoffen, Anästhetika, Keratolytika, Radikalfängern für freie Radikale, Antiseborrhoeika, Antischuppenmitteln, Wirkstoffen gegen Akne und/oder Wirkstoffen, die die Differenzierung und/oder Proliferation und/oder Pigmentierung der Haut vermindern, und den Extrakten pflanzlicher oder bakterieller Herkunft ausgewählt ist.

16. Zusammensetzung nach einem der Ansprüche 12 bis 15, **dadurch gekennzeichnet, daß** sie dazu vorgesehen ist, Störungen zu behandeln, die mit einer Überaktivität der 5α-Reduktase vom Typ 1 zusammenhängen, wobei diese Störungen insbesondere Seborrhoe und/oder Akne und/oder Hirsutismus und/oder androgenetische Alopezie sind.

17. Die Verbindungen der allgemeinen Formel (I') 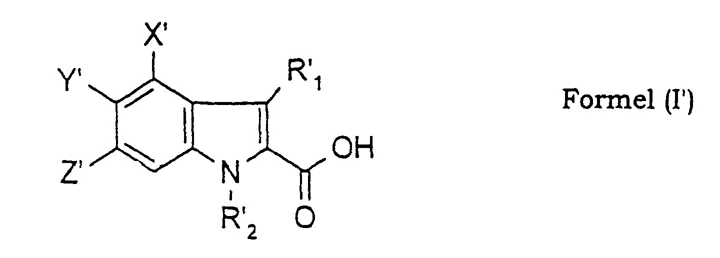 nach den Ansprüchen 6 bis 11 als Arzneimittel.
